(19) Europäisches Patentamt
European Patent Office
Office européen des brevets

(11) **EP 4 268 902 A1**

(12) **EUROPEAN PATENT APPLICATION**
published in accordance with Art. 153(4) EPC

(43) Date of publication:
**01.11.2023 Bulletin 2023/44**

(21) Application number: **21915329.3**

(22) Date of filing: **28.12.2021**

(51) International Patent Classification (IPC):
*A61Q 1/00* (2006.01)        *A61K 8/02* (2006.01)
*A61K 8/44* (2006.01)        *A61K 8/73* (2006.01)

(52) Cooperative Patent Classification (CPC):
**A61K 8/02; A61K 8/44; A61K 8/73; A61Q 1/00**

(86) International application number:
**PCT/JP2021/048957**

(87) International publication number:
**WO 2022/145471 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2020 JP 2020218447**

(71) Applicant: **AJINOMOTO CO., INC.**
**Chuo-ku**
**Tokyo 104-8315 (JP)**

(72) Inventors:
• **YAMAZAKI, Tsuyoshi**
**Kawasaki-shi, Kanagawa 210-8681 (JP)**
• **YAMATO, Naoya**
**Kawasaki-shi, Kanagawa 210-8681 (JP)**
• **BISWAS, Shuvendu**
**Kawasaki-shi, Kanagawa 210-8681 (JP)**
• **KIRYU, Shoichiro**
**Yokkaichi-shi, Mie 510-0885 (JP)**
• **WAKUI, Wataru**
**Kawasaki-shi, Kanagawa 210-8681 (JP)**

(74) Representative: **Mewburn Ellis LLP**
**Aurora Building**
**Counterslip**
**Bristol BS1 6BX (GB)**

(54) **POWDER COMPOSITION FOR COSMETICS OR SKIN TOPICAL AGENT, AND PRODUCTION METHOD THEREFOR**

(57)      The purpose of the present invention is to provide a powder composition that can be used for cosmetics or skin topical agents and that replaces plastic powder bodies such as a nylon powder body, an acrylic powder body, and a urethane powder body. Provided is a composition which is a powder composition containing (a) acyllysine and (b) cellulose and/or starch, and which has a median diameter of the volume-based distribution of (A) acyllysine of 9 μm or less. The contained amount of (A) is 2-40 mass%, and the contained amount of (B) is 3-95 mass%, with respect to 100 mass% of the total of the powder composition. The total amount of (A) and (B) is 10 mass% or more.

EP 4 268 902 A1

**Description**

Technical Field

The present invention relates to a powder composition for cosmetics or skin topical agents, and a method for producing the same.

Background Art

There is a case where plastic powders such as a nylon powder, an acrylic powder, and a urethane powder are blended with cosmetics in order to improve the touch and feel and slipperiness at the time of application. However, in recent years, there have been increasing attentions on environmental loads, and products that can replace these have been demanded. For example, Japanese Unexamined Patent Application Publication No. 2020-97552 describes a microplastic bead-free cosmetic for unevenness compensation, containing (a) 6 to 10% by weight of fumy hydrophobized silica, (b) 5 to 20% by weight of octamethyltrisiloxane and/or decamethyltetrasiloxane, (c) batyl alcohol, (d) 10 to 30% by weight of a powder which does not contain microplastic beads and is different from the (a).

In addition, Japanese Unexamined Patent Application Publication No. 2020-50840 describes a cosmetic blended with porous cellulose particles formed by aggregating specific crystalline cellulose.

In addition, Japanese Unexamined Patent Application Publication No. 2020-152851 describes a cosmetic composition containing specific cellulose derivative particles.

However, there is still a demand for an excellent powder composition that can replace plastic powders such as a nylon powder, an acrylic powder, or a urethane powder.

Citation List

Patent Literatures

Patent Literature 1: Japanese Unexamined Patent Application Publication No. 2020-97552

Patent Literature 2: Japanese Unexamined Patent Application Publication No. 2020-50840

Patent Literature 3: Japanese Unexamined Patent Application Publication No. 2020-152851

Summary of Invention

An object of the present invention is to provide a powder composition that can be used for cosmetics or skin topical agents and that can replace plastic powders such as a nylon powder, an acrylic powder, or a urethane powder.

As a result of conducting earnest studies in view of the above problem, the present inventors have found for the first time that the above-described problem can be solved by a powder composition containing specific acyllysine and cellulose and/or starch in specific blending amounts, and thus completed the present invention. Specifically, the present invention is as follows.

[1] A powder composition comprising:

(A) acyllysine; and
(B) cellulose and/or starch, a median diameter of a volume-based distribution of the (A) acyllysine being 9 μm or less,

wherein

a content of the (A) is 2 to 40% by mass,
a content of the (B) is 3 to 95% by mass, and
a total amount of the (A) and the (B) is 10% by mass or more, in a case where the entire powder composition is considered as 100% by mass.

[2] A powder composition comprising:

(A) acyllysine; and
(B) cellulose and/or starch,

wherein

a content of the (A) is 2 to 40% by mass,
a content of the (B) is 3 to 95% by mass, and
a total amount of the (A) and the (B) is 10% by mass or more, in a case where the entire powder composition is considered as 100% by mass,

and wherein
a water repellent time using an aqueous solution of 35% by mass of ethanol is 30 minutes or more, wherein the water repellent time using the aqueous solution of 35% by mass of ethanol is a time taken for all the powder composition to sediment when 5 g of the aqueous solution of 35% by mass of ethanol is put in a 10-mL glass vial, and 30 mg of the powder composition is disposed on a liquid surface.

[3] A powder composition comprising:

(A) acyllysine; and
(B) cellulose and/or starch,

wherein

a content of the (A) is 2 to 40% by mass,
a content of the (B) is 3 to 95% by mass, and
a total amount of the (A) and (B) is 10% by mass or more, in a case where the entire powder composition is considered as 100% by mass,

and wherein
a water repellent time using an aqueous solution of 30% by mass of ethanol is 60 minutes or more, wherein the water repellent time using the aqueous solution of 30% by mass of ethanol is a time taken for all the powder composition to sediment when 5 g of the aqueous solution of 30% by mass of ethanol is put in a 10-mL glass vial, and 30 mg of the powder composition is disposed on a liquid surface.

[4] The composition according to any one of the above 1 to 3, wherein the (B) contains plate-shaped cellulose.

[5] The composition according to the above 4, comprising 5 to 65% by mass of the plate-shaped cellulose in the case where the entire powder composition is considered as 100% by mass.

[6] The composition according to any one of the above 1 to 5, comprising 5 to 90% by mass of starch in the case where the entire powder composition is considered as 100% by mass.

[7] The composition according to any one of 1 to 6, wherein the (B) contains spherical cellulose.

[8] The composition according to the above 7, comprising 5 to 95% by mass of the spherical cellulose in the case where the entire powder composition is considered as 100% by mass.

[9] The composition according to any one of the above 1 to 8, further comprising (C) silica.

[10] The composition according to the above 9, wherein

the content of the (A) is 2 to 30% by mass,
the content of the (B) is 3 to 65% by mass, and
a content of the (C) is 30 to 90% by mass, in the case where the entire powder composition is considered as 100% by mass.

[11] The composition according to any one of the above 1 to 10, wherein the cellulose is crystalline cellulose.

[12] The composition according to any one of the above 1 to 11, further comprising (D) lysine or a salt thereof.

[13] The composition according to the above 12, comprising a content of the (D) is 0.0001 to 1% by mass in the case where the entire powder composition is considered as 100% by mass.

[14] The composition according to any one of the above 1 to 13, wherein a median diameter of a number-based distribution of the acyllysine is 4 $\mu$m or less.

[15] The composition according to any one of the above 1 to 14, wherein the median diameter of the volume-based distribution of the acyllysine is 6 $\mu$m or less.

[16] The composition according to any one of the above 1 to 15, wherein the median diameter of the number-based distribution of the acyllysine is 2 $\mu$m or less.

[17] The composition according to any one of the above 1 to 16, wherein the median diameter of the volume-based distribution of the acyllysine is 4 $\mu$m or less.

[18] The composition according to any one of the above 1 to 17, wherein a bulk density of the acyllysine is 0.38 g/mL or less.

[19] A cosmetic or skin topical agent comprising the composition according to the above 1 to 18.

[20] A method for producing the composition according to any one of the above 1 to 18, comprising the steps of:

> introducing (A) acyllysine and (B) cellulose and/or starch; and
> mixing.

[21] A method for producing the composition according to any one of claims 9 to 18, comprising the steps of:

> introducing (A) acyllysine, (B) cellulose and/or starch, and (C) silica; and
> mixing.

[22] The production method according to the above 20 or 21, wherein the mixing includes mixing through dry mixing.

[23] The production method according to any one of the above 20 to 22, wherein the mixing includes mixing for 120 minutes or less by using a mixer.

Description of Embodiments

A powder composition of the present invention comprises: (A) acyllysine; and (B) cellulose and/or starch, wherein a median diameter of a volume-based distribution of the (A) acyllysine is 9 $\mu$m or less.

The median diameter of the volume-based distribution of the (A) acyllysine comprised in the powder composition of the present aspect is 9 $\mu$m or less, preferably 6 $\mu$m or less from the viewpoint of improving the adhesion, more preferably 5 $\mu$m or less from the viewpoint of further improving the adhesion and improving the touch and feel, and further preferably 4 $\mu$m or less from the viewpoint of improving the adhesion and shortening the production time.

The median diameter of the number-based distribution of the (A) acyllysine of the present aspect is preferably 4 $\mu$m or less, preferably 3 $\mu$m or less from the viewpoint of improving the adhesion, and further preferably 2 $\mu$m or less from the viewpoint of improving the adhesion and shortening the production time.

The median diameter of the acyllysine can be obtained by measuring the number-based or volume-based particle diameter distribution by using a laser diffraction/scattering particle diameter distribution measuring device. The median diameter means a particle diameter at a point where the distribution curve of the passing particle integrated value % intersects the horizontal axis of 50%. The "number-based distribution" represents a particle diameter distribution calculated based on the number of particles calculated when the frequency of each particle diameter in the particle diameter distribution is calculated, and the "volume-based distribution" represents a particle diameter distribution calculated based on the value that is obtained by calculating the volume of particles on the assumption that each particle is spherical when the frequency of each particle diameter in the particle diameter distribution is calculated.

The bulk density of the (A) acyllysine is preferably 0.38 g/mL or less. Moreover, the bulk density is preferably 0.01 to 0.38 g/mL, more preferably 0.05 to 0.38 g/mL, and further preferably 0.1 to 0.32 g/mL.

The bulk density of the acyllysine can be measured by the following measuring method.

The acyllysine is agitated for 2 minutes or more by using a mixer to be disintegrated, and the bulk density of the crystal thus obtained is measured by using a powder fluidity analyzer (for example, powder rheometer FT-4 (manufactured by Freeman Technology™)). Specifically, after a certain amount of the acyllysine is taken in a holder, conditioning is conducted in accordance with the utilization procedure, and the bulk density is measured from the volume and the mass of the acyllysine after the conditioning in accordance with the following formula.

$$\text{The bulk density} = \text{the mass after the conditioning/the volume after the conditioning (g/mL)}$$

The powder composition of the present invention is also a powder composition comprising: (A) acyllysine; and (B) cellulose and/or starch, wherein a water repellent time using an aqueous solution of 35% by mass of ethanol is 30 minutes or more.

The water repellent time using the aqueous solution of 35% by mass of ethanol is a time taken for all the powder composition to sediment when 5 g of the aqueous solution of 35% by mass of ethanol is put in a 10-mL glass vial and 30 mg of the powder composition is disposed on on a liquid surface. The water repellent time using the aqueous solution of 35% by mass of ethanol is preferably 40 minutes or more, and more preferably 60 minutes or more.

The powder composition of the present invention is also a powder composition comprising: (A) acyllysine; and (B) cellulose and/or starch, wherein a water repellent time using an aqueous solution of 30% by mass of ethanol is 60 minutes or more.

The water repellent time using the aqueous solution of 30% by mass of ethanol is a time taken for all the powder composition to sediment when 5 g of the aqueous solution of 30% by mass of ethanol is put in a 10-mL glass vial, and 30 mg of the powder composition is disposed on a liquid surface.

The method for producing (A) acyllysine having a median diameter of a volume-based distribution of 9 μm or less can be obtained by a method in which a basic solution of acyllysine is added dropwise to a solution such as hydrochloric acid to be crystallized, or a pulverizing method using a machine or the like. Acyllysine having a median diameter of a volume-based distribution of 9 μm or less can be obtained by the method described in WO2020/262367, for example.

The acyl group of the (A) acyllysine is a saturated or unsaturated fatty acid acyl having 8 to 22 carbon atoms, and includes, for example, octanoyl, lauroyl, myristoyl, palmitoyl, stearoyl, octyldodecyl, oleyl, behenyl, acyl of coconut oil fatty acid, acyl of palm kernel oil fatty acid, acyl of beef tallow fatty acid, and the like, but is preferably one or more selected from the group consisting of lauroyl and octanoyl from the viewpoint that they are available in a versatile manner.

The powder composition of the present invention contains (B) cellulose and/or starch, that is, includes an aspect containing (B-1) cellulose, (B-2) starch, or (B-3) cellulose and starch.

The cellulose includes plate-shaped cellulose, spherical cellulose, unshaped cellulose, and the like.

The plate-shaped cellulose includes plate-shaped crystalline cellulose, needle-shaped crystalline cellulose, and the like, and is preferably a plate-shaped crystalline cellulose.

The average particle diameter of the plate-shaped cellulose is preferably 2 to 50 μm, more preferably 5 to 30 μm, and further preferably 8 to 20 μm. Here, the average particle diameter of the plate-shaped cellulose is specified in accordance with JIS (Z8819-2: 2019).

The spherical cellulose includes nonporous cellulose, porous cellulose, and is preferably nonporous cellulose. Here, the spherical shape includes perfectly spherical, substantially spherical, and ellipsoidal shapes.

The average particle diameter of the spherical cellulose is preferably 2 to 30 μm, more preferably 3 to 20 μm, and further preferably 5 to 10 μm. Here, the average particle diameter of the spherical cellulose is specified in accordance with JIS (Z8819-2:2019).

As the cellulose, those registered as crystalline cellulose, cellulose, cellulose acetate, and lignin as INCI names can be used. Cellulose is commercially available, and, for example, TEGO Feel Green produced by Evonik Industries AG, and CELLULOBEADS D-5 and CELLULOBEADS D-10 produced by DAITO KASEI KOGYO CO., LTD. can be used.

The starch includes rice starch, corn starch, potato starch, tapioca starch, pregelatinized starch, partially pregelatinized starch, and the like. The starch is preferably, rice starch, corn starch, or potato starch, and more preferably corn starch. It is preferable to use those registered as rice starch, corn starch, potato starch, and tapioca starch as INCI names.

The average particle diameter of the starch is preferably 5 to 30 μm, more preferably 5 to 25 μm, and further preferably 5 to 20 μm. Here, the average particle diameter of the starch is specified in accordance with JIS (Z8819-2:2019).

Starch is commercially available, and, for example, ST starch P produced by NIPPON STARCH CHEMICAL CO., LTD. can be used.

One aspect of the powder composition of the present invention includes a powder composition comprising: (A) acyllysine; and (B) cellulose. In this aspect, the acyllysine is preferably lauroyl lysine, and more preferably crystal lauroyl lysine. In addition, in this aspect, the cellulose is preferably plate-shaped cellulose or spherical cellulose, and more preferably plate-shaped cellulose.

One aspect of the powder composition of the present invention includes a powder composition comprising: (A) acyllysine; and (B) cellulose and starch. In this aspect, the acyllysine is preferably lauroyl lysine, and more preferably crystal lauroyl lysine. In addition, in this aspect, the cellulose is preferably plate-shaped cellulose or spherical cellulose, and more preferably plate-shaped cellulose. In addition, in this aspect, the starch is preferably corn starch.

The powder composition of the present invention may further comprise (C) silica. As the silica, spherical or unshaped silica can be used, and may be in porous, non-porous, hollow form, or the like. The silica is preferably, nonporous spherical silica, or porous spherical silica, and more preferably porous spherical silica.

The average particle diameter of the silica is preferably 1 to 50 μm, more preferably 3 to 20 μm, and further preferably 5 to 12 μm. Here, the average particle diameter of the silica is specified in accordance with JIS (Z8819-2:2019).

Silica is commercially available, and, for example, porous silica beads SB-300 and SB-700 produced by Miyoshi Kasei Inc., and SUNSPHERE H-31, H-51, H-121, L-31, and L-51 produced by AGC Si-Tech Co., Ltd. can be used.

One aspect of the powder composition of the present invention includes a powder composition comprising: (A) acyllysine; (B) cellulose; and (C) silica. In this aspect, the acyllysine is preferably lauroyl lysine, and more preferably crystal lauroyl lysine. In addition, in this aspect, the cellulose is preferably plate-shaped cellulose or spherical cellulose, and more preferably plate-shaped cellulose. In addition, in this aspect, the silica is preferably, nonporous spherical silica or porous spherical silica, and more preferably porous spherical silica.

One aspect of the powder composition of the present invention includes a powder composition comprising: (A) acyllysine; (B) starch; and (C) silica. In this aspect, the acyllysine is preferably lauroyl lysine, and more preferably crystal lauroyl lysine. In addition, in this aspect, the starch is preferably corn starch. In addition, in this aspect, the silica is preferably, nonporous spherical silica or porous spherical silica, and more preferably porous spherical silica.

The powder composition of the present invention may further comprise (D) lysine or a salt thereof. The salt of lysine includes lysine hydrochloride, which is commercially available, and, for example, lysine hydrochloride produced by Ajinomoto Co., Inc. can be used.

From the viewpoint of obtaining a function replacing a polyamide powder (for example, nylon) or silicone polymer particles (for example, silicone elastomer), preferably, as a first aspect, there is an aspect comprising: (A) acyllysine having a median diameter of a volume-based distribution of 9 $\mu$m or less; (B-1) cellulose; and (C) silica.

From the viewpoint of obtaining a function replacing a polyamide powder (for example, nylon) or silicone polymer particles (for example, silicone elastomer), preferably, as a second aspect, there is an aspect comprising: (A) acyllysine having a median diameter of a volume-based distribution of 9 $\mu$m or less; (B-2) starch; and (C) silica.

From the viewpoint of obtaining a function replacing a polyamide powder (for example, nylon) or silicone polymer particles (for example, silicone elastomer), preferably, as a third aspect, there is an aspect comprising: (A) acyllysine having a median diameter of a volume-based distribution of 9 $\mu$m or less; and (B-3) cellulose and starch.

From the viewpoint of obtaining a function replacing carbon polymer particles (for example, polyacryl·polyethylene·polystyrene), preferably, as a fourth aspect, there is an aspect comprising: (A) acyllysine having a median diameter of a volume-based distribution of 9 $\mu$m or less; (B-2) starch; and (C) silica.

From the viewpoint of obtaining a function replacing carbon polymer particles (for example, polyacryl·polyethylene·polystyrene), preferably, as a fifth aspect, there is an aspect comprising: (A) acyllysine having a median diameter of a volume-based distribution of 9 $\mu$m or less; and (B-3) cellulose and starch.

From the viewpoint of obtaining a function replacing polyurethane particles, preferably, as a sixth aspect, there is an aspect comprising: (A) acyllysine having a median diameter of a volume-based distribution of 9 $\mu$m or less; and (B-1) cellulose. The (B-1) cellulose is preferably spherical cellulose.

From the viewpoint of obtaining a function replacing urethane particles, preferably, as a seventh aspect, there is an aspect comprising: (A) acyllysine having a median diameter of a volume-based distribution of 9 $\mu$m or less; and (B-3) cellulose and starch. The (B-1) cellulose is preferably spherical cellulose.

The content of the (A) acyllysine is 2 to 40% by mass, and preferably 5 to 20% by mass, in the case where the entire powder composition is considered as 100% by mass. If the content of the acyllysine is less than 2% by mass, the water repellency is poor, and if the content of the acyllysine is more than 40% by mass, the adhesiveness to the skin is strong, leading to poor touch and feel.

In the powder composition of the present invention, the total amount of the (A) and the (B) is at least 10% by mass or more in the case where the entire powder composition is considered as 100% by mass. The total amount of the (A) and the (B) is preferably 20% by mass or more, and further preferably 35% by mass or more.

The content of the (B) cellulose and/or starch is 3 to 95% by mass, and preferably 25 to 95% by mass, in the case where the entire powder composition is considered as 100% by mass.

In the aspect in which the (B) contains (B-1) cellulose, the content of the cellulose is preferably 5 to 95% by mass, more preferably 5 to 60% by mass, and further preferably 5 to 40% by mass, in the case where the entire powder composition is considered as 100% by mass. In the case of plate-shaped cellulose, the content of the cellulose is preferably 5 to 95% by mass, more preferably 5 to 60% by mass, and further preferably 5 to 40% by mass. In the case of spherical cellulose, the content of the cellulose is preferably 5 to 95% by mass, more preferably 5 to 60% by mass, and further preferably 5 to 40% by mass.

In the aspect in which the (B) contains (B-2) starch, the content of the starch is preferably 5 to 95% by mass, and more preferably 20 to 90% by mass, in the case where the entire powder composition is considered as 100% by mass.

In the aspect in which the (B) contains (B-3) cellulose and starch, the respective contents of the cellulose and starch are preferably 5 to 75% by mass for cellulose: 20 to 90% by mass for starch, and more preferably 5 to 40% by mass for cellulose: 55 to 90% by mass for starch, in the case where the entire powder composition is considered as 100% by mass.

In the aspect in which the powder composition comprises (C) silica, the content of the (C) is 30 to 90% by mass, and preferably 50 to 80% by mass, in the case where the entire powder composition is considered as 100% by mass.

In the aspect in which the powder composition comprises (D) lysine or a salt thereof, the content of the (D) is 0.0001 to 1% by mass in the case where the entire powder composition is considered as 100% by mass.

The first aspect preferably includes an aspect comprising: (A) 2 to 30% by mass of acyllysine having a median diameter of a volume-based distribution of 9 $\mu$m or less; (B-1) 5 to 40% by mass of cellulose; and (C) 30 to 93% by mass of silica.

The second aspect preferably includes an aspect comprising: (A) 2 to 30% by mass of acyllysine having a median diameter of a volume-based distribution of 9 μm or less; (B-2) 5 to 40% by mass of starch; and (C) 30 to 93% by mass of silica.

The third aspect preferably includes an aspect comprising: (A) 2 to 20% by mass of acyllysine having a median diameter of a volume-based distribution of 9 μm or less; and (B-3) 1 to 38% by mass and 60 to 97% by mass of cellulose and starch, respectively. In the case where the third aspect comprises (C) silica, the third aspect includes an aspect comprising: (A) 2 to 20% by mass of acyllysine having a median diameter of a volume-based distribution of 9 μm or less; (B-3) 5 to 30% by mass and 25 to 90% by mass of cellulose and starch, respectively; and (C) 3 to 78% by mass of silica.

The fourth aspect preferably includes an aspect comprising: (A) 2 to 20% by mass of acyllysine having a median diameter of a volume-based distribution of 9 μm or less; (B-2) 20 to 50% by mass of starch; and (C) 30 to 78% by mass of silica.

The fifth aspect preferably includes an aspect comprising: (A) 2 to 20% by mass of acyllysine having a median diameter of a volume-based distribution of 9 μm or less; and (B-3) 1 to 97% by mass and 1 to 97% by mass of cellulose and starch, respectively.

The sixth aspect preferably includes an aspect comprising: (A) 5 to 40% by mass of acyllysine having a median diameter of a volume-based distribution of 9 μm or less; and (B-1) 60 to 95% by mass of cellulose. The (B-1) cellulose is preferably spherical crystalline cellulose.

The seventh aspect preferably includes an aspect comprising: (A) 5 to 40% by mass of acyllysine having a median diameter of a volume-based distribution of 9 μm or less; and (B-3) 10 to 40% by mass and 50 to 85% by mass of cellulose and starch, respectively. The (B-1) cellulose is preferably spherical crystalline cellulose.

The powder composition of the present invention can be obtained by introducing and mixing the above acyllysine and the above cellulose and/or starch. The acyllysine and the cellulose and/or starch can be introduced simultaneously.

In the case where the powder composition of the present invention comprises (C) silica, the powder composition of the present invention can be obtained by introducing and mixing the above acyllysine, the above cellulose and/or starch, and the above silica. The acyllysine and the cellulose and/or starch as well as the above silica can be introduced simultaneously.

The acyllysine can be mixed through dry mixing. The dry mixing in the present invention includes not only an aspect that does not use a solvent at all, but also an aspect that uses a small amount of a solvent.

In addition, the mixing of the acyllysine can be conducted by mixing for 1 minute or more by using a mixer. The mixing time is preferably 1 minute or more, and more preferably 10 minutes or more. From the viewpoint that the powder composition can be produced at low cost without impairing the productivity, the mixing time is preferably 120 minutes or less. As the mixer, high-speed agitator mixers such as Henschel mixers, household mixers, and high shear mixers; container rotary mixers or container rotary mixers with agitators such as W-type mixers, CV-type mixers, V-type mixers, and rocking mixers; mechanical agitator mixers of ribbon agitating type, multi-shaft paddle type, double-shaft planetary agitating type, conical screw type; compression, shear, and impact type mixers such as airflow agitator mixers, Julia mixers, Nauta mixers, and Nobilta, and the like can be used. From the viewpoint of production at low cost and versatility, a high-speed agitator mixer is preferable.

The composition of the present invention can also be used as a cosmetic or a skin topical agent. The cosmetic or skin topical agent can be prepared in any form that can be applied to, for example, a desired site (e. g., skin, hair, scalp, lips, eye areas, eyelashes, eyelids, nails) in accordance with a conventional method. Cosmetics or topical agents for skin, lips, eyelashes, and nails include, for example, suntan lotions such as sunscreens, body powders, and sprays, makeup cosmetics such as foundations, oshiroi, primers, BB creams, body colors, bronzers, face powders, loose powders, manicures, cheek colors, makeup bases, and concealers, lip cosmetics such as lip colors, lip liners, and lip sticks, eye makeup cosmetics such as eyeliners, eye shadows, eyebrows, and mascaras, leave-on cosmetics such as emulsions, lotions, creams, gels, and leave-on cosmetics, and face masks. Cosmetics or topical agents for hair include, for example, hairdressing agents, hair emulsions, hair treatments, hair conditioners, and hair lotions. Cosmetics or topical agents for scalp include, for example, hair growth agents. Preferable cosmetics include, for example, makeup cosmetics, eye-makeup cosmetics, lip cosmetics, and leave-on cosmetics. Preferable topical agents include, for example, ointments, creams, mousses, and gels. A particularly preferable usage of the present invention includes makeup cosmetics.

In the case of use as a cosmetic or a skin topical agent, the cosmetic can be blended with a component which can be generally used in cosmetics (including pharmaceutical topical agents and quasi-pharmaceutical products) as long as the advantageous effects of the present invention is not impaired. Such a component includes, for example, oil materials, inorganic powders, water, surfactants, amino acids, amino acid derivatives, lower alcohols (for example, ethanol), polyhydric alcohols (for example, glycerol and butylene glycol), sugar alcohols and alkylene oxide adducts thereof, water-soluble polymers (for example, hydroxyethyl cellulose), film formable polymers, gellants (for example, dibutyl lauroyl glutamide and dibutyl ethylhexanoyl glutamide), moisturizers (for example, sodium salt of pyrrolidone carboxylic acid), germicides and antibacterial agents, anti-inflammatory agents, analgesics, antifungal agents, agents for softening or peeling dead skin cells, skin colorants, hormone preparations, ultraviolet absorbers, hair growth agents, antiperspirants and astringent active ingredients (for example, zinc pyrrolidone carboxylate salt), perspiration deodorants, vitamins, blood flow promoters (vasodilators and blood circulation promoters), crude drugs, plant extracts, pH adjusters, chelating agents (for example, EDTA-2Na), viscosity modifiers, pearlescent agents, natural fragrances, synthetic fragrances, dyes and pigments (for example, Red No. 202 and Blue No. 1), antioxidants (for example, tocopherol, tocopherol acetate, and glucosyl pentagallate), antiseptics (for example, methylparaben, butylparaben, propylparaben, and phenoxyethanol), emulsifiers, thickeners, fats and waxes, silicone compounds, balm, and the like.

The oil materials include, for example, higher alcohols such as octyldodecanol and oleyl alcohol; hydrocarbon oils such as squalane, liquid paraffin, hydrogenated polyisobutene, and isododecane; natural or synthetic ester oils such as jojoba seed oil, isononyl isononanoate, isostearyl neopentanoate, cethyl 2-ethylhexanoate, ethylhexyl palmitate, alkyl benzoate, polyglyceryl-2 tetraisostearate, phytosteryl/octyldodecyl lauroyl glutamate, isopropyl lauroyl sarcosinate, ethylhexyl methoxycinnamate, phytosteryl/decyltetradecyl myristoyl methyl beta-alanininate, and glyceryl caprate; diglyceride; natural or synthetic triglycerides such as corn oil, olive oil, sunflower oil, caprylic/capric triglyceride, and triethylhexanoin; high viscous oils such as phytosteryl/isostearyl/cetyl/stearyl/behenyl dimer dilinoleate, diisostearyl malate, hydrogenated polydecene, polyglyceryl-2 triisostearate, polyglyceryl-2 diisostearate, and pentaerythrityl tetraisostearate; silicone oils such as dimethicone, methicone, cyclopentasiloxane, cyclohexasiloxane, phenyl trimethicone, and PEG-10 dimethicone; fluorinated oil materials such as perfluoropolyether, perfluorodecalin, and perfluorooctane; mineral oil; and the like. One of these may be used alone, or two or more of these may be used in combination.

The inorganic powders include, for example, yellow iron oxide, red iron oxide, black iron oxide, fine particles of iron oxide, bismuth oxychloride , zirconium oxide, magnesium oxide, chrome oxide, cobalt oxide, carbon black, ultramarine blue, Prussian blue, zinc oxide, fine particles of zinc oxide, titanium oxide, fine particles of titanium oxide, silica, porous silica, alumina, cerium oxide, boron nitride, calcium sulfate, barium sulfate, magnesium sulfate, calcium carbonate, magnesium carbonate, aluminum silicate, magnesium silicate, silicon carbide, dye, lake, sericite, mica, talc, kaolin, clay, bentonite, plate-shaped barium sulfate, butterfly-shaped barium sulfate, hydroxyapatite, and the like. One of these may be used alone, or two or more of these may be used in combination. Furthermore, the inorganic powder may be a complex of those mentioned above (for example, silica-coated titanium dioxide, mica-coated titanium dioxide, and titanium-coated mica), and may be those obtained by applying a surface treatment such as a silicone treatment, a fluorine compound treatment, a silane coupling agent treatment, a silane treatment, an organic titanate treatment, a fatty acid treatment (for example, a stearoylglutamic acid treatment), a metal soap treatment (for example, an aluminum stearate treatment), an oil material treatment, and an amino acid treatment to those mentioned above (for example, silicone-treated talc, silicone-treated mica, silicone-treated sericite, silicone-treated titanium dioxide, silicone-treated red iron oxide, silicone-treated yellow iron oxide, silicone-treated black iron oxide, stearoylglutamic acid-treated titanium dioxide, stearoylglutamic acid-treated yellow iron oxide, stearoylglutamic acid-treated red iron oxide, stearoylglutamic acid-treated black iron oxide, aluminum stearate-treated titanium dioxide, and the like).

Examples

Method for Measuring Various Particle Diameters of Acyllysin:

A particle diameter distribution was measured from a volume cumulative value by using a laser diffraction/scattering particle diameter distribution measuring device (Partica LA-950 manufactured by HORIBA). Results obtained by the measurement were analyzed by using software attached to the device to obtain various particle diameters of number-based distribution and volume-based distribution. 20 mg of acyllysine was added to 5 g of isopropyl alcohol, and was exposed to an ultrasonic wave while agitating for 30 minutes to be disintegrated and dispersed by using an ultrasonic wave device having an output of 300 Watt. An appropriate amount of this dispersion liquid was added to 500 mL of isopropyl alcohol in accordance with the procedure of the device, and a dispersion sample having an appropriate concentration was prepared while checking the transparency. While this sample was being circulated at a flow rate of 10 mL/min, an ultrasonic wave was applied to the sample for 30 minutes to disperse the sample into primary particles. After deaeration, the particle diameter distribution and various particle diameters of acyllysine in the sample were obtained by using a flow cell.

Method for Measuring Bulk Density of Acyllysine:

Lauroyl lysine was agitated for 2 minutes or more by using a mixer to be disintegrated. The bulk density of the crystal thus obtained was measured by using powder rheometer FT-4 (manufactured by Freeman Technology™). Specifically, a certain amount of lauroyl lysine was measured and taken in a holder, and then conditioning was conducted in accordance with the utilization procedure. From the volume and mass of lauroyl lysine after the conditioning, the bulk density was measured in accordance with the following formula.

The bulk density=the mass after the conditioning/the volume after the conditioning (g/mL)

Preparation of Acyllysine

(Acyllysine of Preparation Example A)

After 5.7 g of sodium hydroxide was dissolved into a mixed solution of 97.4 g of methanol and 62.7 g of water at room temperature, the mixture was heated to about 50°C. Then, 36.4 g of a commercially-available Nε-lauroyl lysine crystal was added thereto, and was dissolved at the same temperature to obtain a solution of Nε-lauroyl lysine. Thereafter, the solution of Nε-lauroyl lysine was added dropwise over 75 minutes into an aqueous solution of hydrochloric acid (600 mL) having a concentration of 0.1 mol/L, which was kept being cooled to 30°C or less while the pH was maintained at 0.7 to 1.3. After the completion of the dropwise addition, the pH was adjusted to 7.0 with sodium hydroxide, and the prepared crystal was filtrated and dried under reduced pressure to obtain 36.0 g of a white crystal. The median diameter of the volume-based distribution of the crystal thus obtained was 9 μm.

Note that Nε-lauroyl lysine having a smaller median diameter of the volume-based distribution can be obtained by lowering the temperature of the aqueous solution of hydrochloric acid to which the solution of Nε-lauroyl lysine is added dropwise.

(Acyllysine of Preparation Example 1)

A white crystal of Nε-lauroyl lysine having a median diameter of the volume-based distribution of 3.9 μm, a median diameter of the number-based distribution of 1.1 μm, and a bulk density of 0.28 g/mL was obtained by appropriately changing the temperature of the aqueous solution of hydrochloric acid to which the solution of Nε-lauroyl lysine was added dropwise, in accordance with Preparation Example A.

(Acyllysine of Preparation Example 2)

A white crystal of Nε-lauroyl lysine having a median diameter of the volume-based distribution of 4.4 μm was obtained by appropriately changing the temperature of the aqueous solution of hydrochloric acid to which the solution of Nε-lauroyl lysine was added dropwise, in accordance with Preparation Example A.

(Acyllysine of Preparation Example 3)

A white crystal of Nε-lauroyl lysine having a median diameter of the volume-based distribution of 5.7 μm and a median diameter of the number-based distribution of 3.5 μm was obtained by appropriately changing the temperature of the aqueous solution of hydrochloric acid to which the solution of Nε-lauroyl lysine was added dropwise, in accordance with Preparation Example A.

(Acyllysine of Preparation Example 4)

A white crystal of Nε-lauroyl lysine having a median diameter of the volume-based distribution of 6.1 μm was obtained by appropriately changing the temperature of the aqueous solution of hydrochloric acid to which the solution of Nε-lauroyl lysine was added dropwise, in accordance with Preparation Example A.

(Acyllysine of Preparation Example 5)

A white crystal of Nε-lauroyl lysine having a median diameter of the volume-based distribution of 9.0 μm was obtained by appropriately changing the temperature of the aqueous solution of hydrochloric acid to which the solution of Nε-lauroyl lysine was added dropwise, in accordance with Preparation Example A.

(Acyllysine of Preparation Example 6)

Nε-lauroyl lysine was obtained by milling commercially-available Nε-lauroyl lysine (produced by Ajinomoto Co., Inc. under the trade name of AMIHOPE LL) by using Picoline (swirling jet mill) of Hosokawa Micron Corporation with a supply gas pressure of 0.60 MPa, a milling gas pressure of 0.60 MPa, and a supply rate of 49.3 g/h. The median diameter of the volume-based distribution of the Nε-lauroyl lysine thus obtained was 3.2 μm.

(Acyllysine of Preparation Example 7)

A white crystal of Nε-lauroyl lysine having a median diameter of the volume-based distribution of 2.2 μm was obtained by appropriately changing the temperature of the aqueous solution of hydrochloric acid to which the solution of Nε-lauroyl lysine was added dropwise, in accordance with Preparation Example A.

(Acyllysine of Preparation Example 8)

A white crystal was obtained in accordance with Production Example 4 of WO01/014317. As a result of conducting various measurements on the crystal thus obtained, the median diameter of the volume-based distribution of the obtained Nε-lauroyl lysine was 14 μm.

Sensory Evaluation Method:

Hearing investigations were conducted to 5 expert panelists for 5 evaluation items, namely, smoothness, moistness, ball-bearing effect, adhesiveness to the skin, and softness. The score of Comparison Control was deemed as 5, and a score was given to each of the evaluation items in accordance with the following methods, and the score of the evaluation item was determined with an average score of the 5 panelists. Furthermore, evaluation was determined in accordance with a 5-scale of A to E with an average value of the scores of each evaluation item.

(Score): (Evaluation)

6: Very Good
5: Good
4: Slightly Good
3: Slightly Poor
2: Poor
1: Very Poor

(Determination):(Score Average Value)

A: 5.0 or more
B: 4.4 to 4.9
C: 3.9 to 4.3
D: 3.1 to 3.8
E: 3.0 or less

(Examples 1-1 to 1-7 and Comparative Examples 1-1 to 1-2)

Raw materials were weighed in a blending ratio (% by mass) shown in Table 1 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when nylon (produced by Toray Industries, Inc., SP-500) was used as Comparison Control are shown in Table 1. The score of each evaluation item is shown in Table 2-1.

Table 1

| | Example 1-1 | Example 1-2 | Example 1-3 | Example 1-4 | Example 1-5 | Example 1-6 | Example 1-7 | Comparative Example 1-1 | Comparative Example 1-2 |
|---|---|---|---|---|---|---|---|---|---|
| Acyllysine of Preparation Example 1 | 10 | | | | | | | | |
| Acyllysine of Preparation Example 2 | | 10 | | | | | | | |
| Acyllysine of Preparation Example 3 | | | 10 | | | | | | |
| Acyllysine of Preparation Example 4 | | | | 10 | | | | | |
| Acyllysine of Preparation Example 5 | | | | | 10 | | | | |
| Acyllysine of Preparation Example 6 | | | | | | 10 | | | |
| Acyllysine of Preparation Example 7 | | | | | | | 10 | | |
| Acyllysine of Preparation Example 8 | | | | | | | | 10 | |
| Acyllysine of Comparative Example *4 | | | | | | | | | 10 |
| Porous spherical silica *1 | 60 | 60 | 60 | 60 | 60 | 60 | 60 | 60 | 60 |
| Plate-shaped cellulose *2 | 30 | 30 | 30 | 30 | 30 | 30 | 30 | 30 | 30 |
| Sensory evaluation | A | B | B | C | C | A | A | E | E |

11

[Table 2-1]

|  | Smoothness | Moistness | Ball-bearing effect | Adhesiveness to the skin | Softness | Total |
|---|---|---|---|---|---|---|
| Example 1-1 | 4.6 | 5.6 | 5.2 | 5.8 | 5.0 | 5.2 |
| Example 1-2 | 4.0 | 5.4 | 4.8 | 5.4 | 5.0 | 4.9 |
| Example 1-3 | 3.6 | 5.2 | 4.8 | 5.0 | 4.6 | 4.6 |
| Example 1-4 | 3.6 | 4.8 | 4.4 | 4.8 | 4.0 | 4.3 |
| Example 1-5 | 3.5 | 4.6 | 4.0 | 4.7 | 4.0 | 4.2 |
| Example 1-6 | 4.7 | 5.4 | 5.2 | 5.8 | 5.0 | 5.2 |
| Example 1-7 | 4.6 | 5.6 | 5.4 | 5.8 | 5.0 | 5.3 |
| Comparative Example 1-1 | 3.4 | 3.0 | 2.1 | 3.3 | 3.1 | 3.0 |
| Comparative Example 1-2 | 3.4 | 2.8 | 2.0 | 3.2 | 3.2 | 2.9 |

A water repellent time was evaluated for the evaluation samples. The evaluation method was such that 5 g of an aqueous solution of 30% by mass or 35% by mass of ethanol was put in a 10-mL glass vial, and 30 mg of each evaluation sample was gradually introduced from above, and the time for which the evaluation sample remained on the liquid surface was measured as the water repellent time. Evaluation results are shown in Table 2-2.

Table 2-2

| | Example 1-7 | Example 1-6 | Example 1-1 | Example 1-2 | Example 1-3 | Example 1-4 | Example 1-5 | Comparative Example 1-2 |
|---|---|---|---|---|---|---|---|---|
| Median diameter of the volume-based distribution of acyllysine ($\mu$m) | 2.2 | 3.2 | 3.9 | 4.4 | 5.7 | 6.1 | 9.0 | 20 |
| Water repellent time (35% ethanol) | 60 minutes or more | 60 minutes or more | 60 minutes or more | 60 minutes or more | 43 minutes | 43 minutes | 38 minutes | 5 minutes |
| Water repellent time (30% ethanol) | 60 minutes or more | 60 minutes or more | 60 minutes or more | 60 minutes or more | 60 minutes or more | 60 minutes or more | 60 minutes or more | 9 minutes |

# EP 4 268 902 A1

(Examples 2-1 to 2-12 and Comparative Examples 2-1 to 2-4)

Raw materials were weighed in a blending ratio (% by mass) shown in Table 3-1 to Table 3-3 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when nylon (produced by Toray Industries, Inc., SP-500) was used as Comparison Control are shown in Table 3-1 to Table 3-3.

Table 3-1

|  | Example 2-1 | Example 2-2 | Example 2-3 | Example 2-4 | Example 2-5 |
|---|---|---|---|---|---|
| Acyllysine of Preparation Example 1 | 8 | 3 | 30 | 8 | 8 |
| Plate-shaped cellulose *2 | 28 | 12 | 13 | 5 | 62 |
| Starch *5 |  |  |  |  |  |
| Porous spherical silica * 1 | 64 | 85 | 57 | 87 | 30 |
| Sensory evaluation | A | A | A | A | A |

Table 3-2

|  | Example 2-6 | Example 2-7 | Example 2-8 | Example 2-9 | Example 2-10 |
|---|---|---|---|---|---|
| Acyllysine of Preparation Example 1 | 8 | 8 | 9 | 5 | 2 |
| Plate-shaped cellulose *2 | 82 |  |  |  |  |
| Starch *5 |  | 5 | 27 | 40 | 83 |
| Porous spherical silica * 1 | 10 | 87 | 64 | 55 | 15 |
| Sensory evaluation | C | B | A | B | C |

Table 3-3

|  | Example 2-11 | Example 2-12 | Comparative Example 2-1 | Comparative Example 2-2 | Comparative Example 2-3 | Comparative Example 2-4 |
|---|---|---|---|---|---|---|
| Acyllysine of Preparation Example 1 | 2 | 2 | 8 | 1 | 1 | 8 |
| Plate-shaped cellulose *2 | 9 | 12 | 1 | 9 | 5 |  |
| Starch *5 |  |  |  |  | 94 | 2 |
| Porous spherical silica * 1 | 89 | 86 | 91 | 90 |  | 90 |
| Sensory evaluation | C | B | D | D | D | D |

(Examples 3-1 to 3-3)

Raw materials were weighed in a blending ratio (% by mass) shown in Table 4 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when nylon (produced by Toray Industries, Inc., SP-500) was used as Comparison Control are shown in Table 4.

Table 4

| | Example 2-1 | Example 3-1 | Example 3-2 | Example 3-3 |
|---|---|---|---|---|
| Acyllysine of Preparation Example 1 | 8 | 8 | 8 | 8 |
| Plate-shaped cellulose *2 | 28 | 28 | 28 | 28 |
| Starch *5 | | | | |
| Porous spherical silica * 1 | 64 | | | |
| Porous spherical silica *6 | | 64 | | |
| Nonporous spherical silica *7 | | | 64 | |
| Nonporous spherical silica *8 | | | | 64 |
| Sensory evaluation | A | B | A | A |

(Examples 4-1 to 4-11)

Raw materials were weighed in a blending ratio (% by mass) shown in Table 5-1 and Table 5-2 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when nylon (produced by Toray Industries, Inc., SP-500) was used as Comparison Control are shown in Tables 5-1 and 5-2.

Table 5-1

| | Example 2-1 | Example 4-1 | Example 4-2 | Example 4-3 | Example 4-4 | Example 4-5 |
|---|---|---|---|---|---|---|
| Acyllysine of Preparation Example 1 | 8 | 8 | 8 | 8 | 8 | 8 |
| Plate-shaped cellulose *2 | 28 | | | | | 5 |
| Plate-shaped cellulose *9 | | 28 | | | | |
| Plate-shaped cellulose *10 | | | 28 | | | |
| Spherical cellulose * 11 | | | | 28 | | |
| Spherical cellulose *12 | | | | | 28 | |
| Starch *5 | | | | | | 87 |
| Porous spherical silica *1 | 64 | 64 | 64 | 64 | 64 | |
| Sensory evaluation | A | A | A | A | A | A |

Table 5-2

|  | Example 4-6 | Example 4-7 | Example 4-8 | Example 4-9 | Example 4-10 | Example 4-11 |
|---|---|---|---|---|---|---|
| Acyllysine of Preparation Example 1 | 8 | 8 | 8 | 8 | 8 | 8 |
| Plate-shaped cellulose *2 |  |  |  |  | 1 |  |
| Plate-shaped cellulose *9 | 5 |  |  |  |  |  |
| Plate-shaped cellulose *10 |  | 5 |  |  |  |  |
| Spherical cellulose *11 |  |  | 5 |  |  |  |
| Spherical cellulose *12 |  |  |  | 5 |  | 1 |
| Starch *5 | 87 | 87 | 87 | 87 | 91 | 91 |
| Porous spherical silica *1 |  |  |  |  |  |  |
| Sensory evaluation | A | A | A | A | B | B |

(Examples 5-1 to 5-4)

Raw materials were weighed in a blending ratio (% by mass) shown in Table 6-1 and Table 6-2 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when PMMA particles (produced by Matsumoto Yushi-Seiyaku Co., Ltd.: Matsumoto Microsphere M-100) were used as Comparison Control are shown in Table 6-1 and Table 6-2.

Table 6-1

|  | Example 2-8 | Example 5-1 | Example 5-2 |
|---|---|---|---|
| Acyllysine of Preparation Example 1 | 9 | 9 | 9 |
| Plate-shaped cellulose *2 |  |  |  |
| Starch *5 | 27 |  |  |
| Starch *14 |  | 27 |  |
| Starch *15 |  |  | 27 |
| Porous spherical silica *1 | 64 | 64 | 64 |
| Sensory evaluation | A | A | A |

Table 6-2

|  | Example 4-5 | Example 5-3 | Example 5-4 |
|---|---|---|---|
| Acyllysine of Preparation Example 1 | 8 | 8 | 8 |
| Plate-shaped cellulose *2 | 5 | 5 | 5 |

(continued)

|  | Example 4-5 | Example 5-3 | Example 5-4 |
|---|---|---|---|
| Starch *5 | 87 | | |
| Starch *14 | | 87 | |
| Starch *15 | | | 87 |
| Porous spherical silica *1 | | | |
| Sensory evaluation | A | A | A |

(Examples 6-1 to 6-14)

Raw materials were weighed in a blending ratio (% by mass) shown in Table 7-1 to Table 7-3 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes). Evaluation results when urethane particles (produced by DAINICHISEIKA COLOR & CHEM MFG CO., LTD.: DAIMIC BEAZ) were used as Comparison Control are shown in Table 7-1 to Table 7-3.

Table 7-1

|  | Example 6-1 | Example 6-2 | Example 6-3 | Example 6-4 |
|---|---|---|---|---|
| Acyllysine of Preparation Example 1 | 5 | 10 | 15 | 30 |
| Plate-shaped cellulose *2 | | | | |
| Spherical cellulose *12 | 95 | 90 | 85 | 70 |
| Starch *5 | | | | |
| Sensory evaluation | A | A | A | A |

Table 7-2

|  | Example 6-5 | Example 6-6 | Example 6-7 | Example 6-8 | Example 6-9 |
|---|---|---|---|---|---|
| Acyllysine of Preparation Example 1 | 15 | 15 | 15 | 15 | 15 |
| Plate-shaped cellulose *2 | 85 | 35 | 55 | | |
| Spherical cellulose *12 | | 50 | 30 | 30 | 15 |
| Starch *5 | | | | 55 | 70 |
| Sensory evaluation | C | B | C | A | A |

Table 7-3

|  | Example 6-10 | Example 6-11 | Example 6-12 | Example 6-13 | Example 6-14 |
|---|---|---|---|---|---|
| Acyllysine of Preparation Example 1 | 10 | 5 | 15 | 5 | 2 |
| Plate-shaped cellulose *2 | | | 34 | | |
| Spherical cellulose *12 | 15 | 15 | 50 | 5 | 5 |
| Starch *5 | 75 | 80 | | 90 | 93 |
| Lysine hydrochloride | | | 1 | | |
| Sensory evaluation | A | B | A | B | C |

(Examples 7-1 to 7-4)

Raw materials were weighed in a blending ratio (% by mass) shown in Table 8 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes).

The non-dryness at the time of applying various mixtures was evaluated by 5 expert panelists in accordance with the following criteria.

Method for Evaluating Dryness at the Time of Application:

1) There is no feeling of dryness at the time of application.... 4 points
2) There hardly is feeling of dryness at the time of application.... 3 points
3) There is little feeling of dryness at the time of application.... 2 points
4) There is feeling of dryness at the time of application.... 1 point

Determination was made as follows based on average scores of evaluation of the 5 expert panelists. Results are shown in Table 14.

Average score of evaluation 3.5 or more: Very favorable (A)
Average score of evaluation 2.5 or more and less than 3.5: Slightly favorable (B)
Average score of evaluation 1.5 or more and less than 2.5: Not so favorable (C)
Average score of evaluation and less than 1.5: Not favorable at all (D)

Table 8

|  | Example 7-1 | Example 7-2 | Example 7-3 | Example 7-4 |
|---|---|---|---|---|
| Acyllysine of Preparation Example 1 | 8 | 8 | 8 | 8 |
| Plate-shaped cellulose *2 |  |  | 5 | 5 |
| Starch *5 | 5 | 5 | 87 | 86.99 |
| Spherical silica * 1 | 87 | 86.5 |  |  |
| Lysine hydrochloride |  | 0.5 |  | 0.01 |
| Dryness at the time of application | B | A | B | A |

Raw materials: Raw materials used in Examples and Comparative Examples shown in Table 1 to Table 8 described above are as follows.
*1 produced by AGC Si-Tech Co., Ltd.: average particle diameter 5 $\mu$m
*2 produced by Evonik Operations (TEGO Feel): average particle diameter 30 $\mu$m
*4 produced by Ajinomoto Co., Inc., AMIHOPE LL: median diameter of the volume-based distribution 20 $\mu$m
*5 produced by NIPPON STARCH CHEMICAL CO., LTD. (ST starch C(W)): average particle diameter 18 $\mu$m
*6 produced by AGC Si-Tech Co., Ltd.: average particle diameter 12 $\mu$m
*7 produced by AGC Si-Tech Co., Ltd.: average particle diameter 4 $\mu$m
*8 produced by AGC Si-Tech Co., Ltd.: average particle diameter 10 $\mu$m
*9 produced by Evonik Operations: average particle diameter 10 $\mu$m
*10 average particle diameter: 20 $\mu$m, bulk density 0.22 g/cm$^3$
*11 produced by DAITO KASEI KOGYO CO., LTD.: average particle diameter 5 $\mu$m
*12 produced by DAITO KASEI KOGYO CO., LTD.: average particle diameter 10 $\mu$m
*14 produced by DAITO KASEI KOGYO CO., LTD. (RICE PW): average particle diameter 8 $\mu$m
*15 produced by NIPPON STARCH CHEMICAL CO., LTD. (ST starch P): average particle diameter 32 $\mu$m

(Production Examples 1 to 4)

Production Examples of the powder composition of the present invention are shown below.

(Production Example 1)

By using a Henschel mixer (Nippon Coke & Engineering Co., Ltd. FM mixer FM150), 15.0 kg of silica, 7.5 kg of crystalline cellulose, and 2.5 kg of Nε-lauroyl lysine (having a median diameter of the volume-based distribution of 3.9 μm) are weighed, and simultaneously introduced into the mixer (the total amount of the powder: 25 kg). The powder is mixed with the circumferential speed of the agitating blade being 60 m/sec for 30 minutes.

(Production Example 2)

By using a agitating and mixing granulator (Powrex Corp. Vertical Granulator VG-200), 15.0 kg of silica, 7.5 kg of crystalline cellulose, and 2.5 kg of Nε-lauroyl lysine (having a median diameter of the volume-based distribution of 3.9 μm) are weighed, and simultaneously introduced into the mixer (the total amount of the powder: 25 kg). The powder is mixed with a blade rotational speed of: 100 rpm and a cross-screw rotational speed of: 1800 rpm for a mixing time of 60 minutes.

(Production Example 3)

By using a agitating and mixing granulator (EARTHTECHNICA Co., Ltd. high speed mixer LFS2), 120 g of silica, 60 g of crystalline cellulose, and 20 g of lauroyl lysine (having a median diameter of the volume-based distribution of 3.9 μm) are weighed, and simultaneously introduced into the mixer (the total amount of the powder: 200 g). The powder is mixed with a blade rotational speed of: 500 rpm and a chopper rotational speed of: 2000 rpm for a mixing time of 60 minutes.

(Production Example 4)

By using a agitating and mixing granulator (EARTHTECHNICA Co., Ltd. high speed mixer LFS2), 120 g of silica, 60 g of crystalline cellulose, and 20 g of octanoyl lysine (having a median diameter of the volume-based distribution of 8.8 μm) are weighed, and simultaneously introduced into the mixer (the total amount of the powder: 200 g). The powder is mixed with a blade rotational speed of: 500 rpm and a chopper rotational speed of: 2000 rpm for a mixing time of 60 minutes.

(Evaluation Using Pressed Foundation)

As cosmetics or skin topical agents using the powder compositions of the present invention, pressed foundations were prepared, and were evaluated.
Regarding the evaluation on the usability, 5 expert panelists gave scores to 4 evaluation items, namely, "adhesion", "smoothness on application", "non-stickiness", and "moisturization", and the score of each evaluation item was determined using an average score of the 5 panelists. Furthermore, evaluation was determined in accordance with a 5-scale of A to D with an average value of the scores of each evaluation item.

(Score): (Evaluation)

6: Very Good
5: Good
4: Slightly Good
3: Slightly Poor
2: Poor
1: Very Poor

(Determination):(Score average value)

A: 5 points or more
B: 4 points or more and less than 5 points
C: 3 points or more and less than 4 points

D: less than 3 points

(Examples 8)

Pressed foundations were prepared by using components shown in Table 9-1 and Table 9-2 as follows. B was heated and dissolved. A was weighed and mixed by using Labo Milser for 30 seconds. B was added to A, and further agitated by using Labo Mixer for 5 minutes, and mixed 30 seconds × three times by using Labo Milser. The mixture was sieved by using a 150-μm opening sieve to make the particle diameters uniform, and the mixture was then packed in a gold plate and compression-molded.

Table 9-1

| | | Comparative Example 8-1 | Comparative Example 8-2 | Comparative Example 8-3 | Example 8-1 | Example 8-2 | Example 8-3 | Example 8-4 | Example 8-5 |
|---|---|---|---|---|---|---|---|---|---|
| | Example No. of the powder composition of the present invention described below | | | Comparative Example 2-2 | Example 2-1 | Example 2-4 | Example 2-5 | Example 2-7 | Example 2-8 |
| | | (wt%) | (wt%) | (wt%) | (wt%) | (wt%) | (wt%) | (wt%) | (wt%) |
| A | Talc, dimethicone *1 | 20.40 | 20.40 | 20.40 | 20.40 | 20.40 | 20.40 | 20.40 | 20.40 |
| | Mica, dimethicone *2 | 30.00 | 30.00 | 30.00 | 30.00 | 30.00 | 30.00 | 30.00 | 30.00 |
| | Mica, dimethicone *3 | 20.00 | 20.00 | 20.00 | 20.00 | 20.00 | 20.00 | 20.00 | 20.00 |
| | Titanium oxide, dimethicone *4 | 8.00 | 8.00 | 8.00 | 8.00 | 8.00 | 8.00 | 8.00 | 8.00 |
| | Titanium oxide, Al stearate *5 | 3.00 | 3.00 | 3.00 | 3.00 | 3.00 | 3.00 | 3.00 | 3.00 |
| | Zinc oxide, methicone *6 | 2.00 | 2.00 | 2.00 | 2.00 | 2.00 | 2.00 | 2.00 | 2.00 |
| | Red iron oxide, dimethicone *7 | 0.40 | 0.40 | 0.40 | 0.40 | 0.40 | 0.40 | 0.40 | 0.40 |
| | Yellow iron oxide, dimethicone *8 | 1.00 | 1.00 | 1.00 | 1.00 | 1.00 | 1.00 | 1.00 | 1.00 |
| | Black iron oxide, dimethicone *9 | 0.15 | 0.15 | 0.15 | 0.15 | 0.15 | 0.15 | 0.15 | 0.15 |
| | Nylon-12 *10 | 4.00 | - | - | - | - | - | - | - |
| | PMMA particles | - | 4.00 | - | - | - | - | - | - |
| | Urethane particles | - | - | - | - | - | - | - | - |
| | The powder composition of the present invention | - | - | 4.00 | 4.00 | 4.00 | 4.00 | 4.00 | 4.00 |
| | Methylparaben | 0.05 | 0.05 | 0.05 | 0.05 | 0.05 | 0.05 | 0.05 | 0.05 |
| B | Dimethicone *11 | 4.98 | 4.98 | 4.98 | 4.98 | 4.98 | 4.98 | 4.98 | 4.98 |
| | Polyglyceryl-2 tetraisostearate *12 | 3.00 | 3.00 | 3.00 | 3.00 | 3.00 | 3.00 | 3.00 | 3.00 |
| | Dipentaerythrityl hexahydroxystearate/ hexastearate/hexarosinate *13 | 1.00 | 1.00 | 1.00 | 1.00 | 1.00 | 1.00 | 1.00 | 1.00 |
| | Mineral oil *14 | 2.00 | 2.00 | 2.00 | 2.00 | 2.00 | 2.00 | 2.00 | 2.00 |
| | Methylparaben | 0.01 | 0.01 | 0.01 | 0.01 | 0.01 | 0.01 | 0.01 | 0.01 |
| | Tocopherol | 0.01 | 0.01 | 0.01 | 0.01 | 0.01 | 0.01 | 0.01 | 0.01 |

EP 4 268 902 A1

21

(continued)

| | Comparative Example 8-1 | Comparative Example 8-2 | Comparative Example 8-3 | Example 8-1 | Example 8-2 | Example 8-3 | Example 8-4 | Example 8-5 |
|---|---|---|---|---|---|---|---|---|
| Example No. of the powder composition of the present invention described below | | | Comparative Example 2-2 | Example 2-1 | Example 2-4 | Example 2-5 | Example 2-7 | Example 2-8 |
| Total | 100.0 | 100.0 | 100.0 | 100.0 | 100.0 | 100.0 | 100.0 | 100.0 |
| Sensory evaluation | A | A | C | A | A | A | B | A |

Table 9-2

| | | Example 8-6 | Example 8-7 | Example 8-8 | Example 8-9 | Example 8-10 | Example 8-11 | Example 8-12 |
|---|---|---|---|---|---|---|---|---|
| | Example No. of the powder composition of the present invention described below | Example 2-11 | Example 3-1 | Example 3-3 | Example 4-1 | Example 4-4 | Example 4-10 | Example 5-1 |
| | | (wt%) | (wt%) | (wt%) | (wt%) | (wt%) | (wt%) | (wt%) |
| A | Talc, dimethicone *1 | 20.40 | 20.40 | 20.40 | 20.40 | 20.40 | 20.40 | 20.40 |
| | Mica, dimethicone *2 | 30.00 | 30.00 | 30.00 | 30.00 | 30.00 | 30.00 | 30.00 |
| | Mica, dimethicone *3 | 20.00 | 20.00 | 20.00 | 20.00 | 20.00 | 20.00 | 20.00 |
| | Titanium oxide, dimethicone *4 | 8.00 | 8.00 | 8.00 | 8.00 | 8.00 | 8.00 | 8.00 |
| | Titanium oxide, Al stearate *5 | 3.00 | 3.00 | 3.00 | 3.00 | 3.00 | 3.00 | 3.00 |
| | Zinc oxide, methicone *6 | 2.00 | 2.00 | 2.00 | 2.00 | 2.00 | 2.00 | 2.00 |
| | Red iron oxide, dimethicone *7 | 0.40 | 0.40 | 0.40 | 0.40 | 0.40 | 0.40 | 0.40 |
| | Yellow iron oxide, dimethicone *8 | 1.00 | 1.00 | 1.00 | 1.00 | 1.00 | 1.00 | 1.00 |
| | Black iron oxide, dimethicone *9 | 0.15 | 0.15 | 0.15 | 0.15 | 0.15 | 0.15 | 0.15 |
| | Nylon-12 *10 | - | - | - | - | - | - | - |
| | PMMA particles | - | - | - | - | - | - | - |
| | Urethane particles | - | - | - | - | - | - | - |
| | The powder composition of the present invention | 4.00 | 4.00 | 4.00 | 4.00 | 4.00 | 4.00 | 4.00 |
| | Methylparaben | 0.05 | 0.05 | 0.05 | 0.05 | 0.05 | 0.05 | 0.05 |
| B | Dimethicone *11 | 4.98 | 4.98 | 4.98 | 4.98 | 4.98 | 4.98 | 4.98 |
| | Polyglyceryl-2 tetraisostearate *12 | 3.00 | 3.00 | 3.00 | 3.00 | 3.00 | 3.00 | 3.00 |
| | Dipentaerythrityl hexahydroxystearate/ hexastearate/hexarosinate *13 | 1.00 | 1.00 | 1.00 | 1.00 | 1.00 | 1.00 | 1.00 |
| | Mineral oil *14 | 2.00 | 2.00 | 2.00 | 2.00 | 2.00 | 2.00 | 2.00 |
| | Methylparaben | 0.01 | 0.01 | 0.01 | 0.01 | 0.01 | 0.01 | 0.01 |
| | Tocopherol | 0.01 | 0.01 | 0.01 | 0.01 | 0.01 | 0.01 | 0.01 |
| | Total | 100.0 | 100.0 | 100.0 | 100.0 | 100.0 | 100.0 | 100.0 |

(continued)

| | | Example 8-6 | Example 8-7 | Example 8-8 | Example 8-9 | Example 8-10 | Example 8-11 | Example 8-12 |
|---|---|---|---|---|---|---|---|---|
| | Example No. of the powder composition of the present invention described below | Example 2-11 | Example 3-1 | Example 3-3 | Example 4-1 | Example 4-4 | Example 4-10 | Example 5-1 |
| | Sensory evaluation | A | B | A | A | A | B | A |

Raw materials: Raw materials used in Examples and Comparative Examples shown in Table 9-1 to Table 9-2 described above are as follows.

*1 SA-Talc JA-46R, produced by Miyoshi Kasei Inc.

*2 SA-Sericite FSE, produced by Miyoshi Kasei Inc.

*3 SA-Mica Y-2300, produced by Miyoshi Kasei Inc.

*4 SA-Ttitanium CR50 (100%), produced by Miyoshi Kasei Inc.

*5 MT-100Z, produced by Tayca Corporation

*6 MZ-303S, produced by Tayca Corporation

*7 SA-Red R-516PS (100%), produced by Miyoshi Kasei Inc.

*8 SA-Yellow LL-100P (100%), produced by Miyoshi Kasei Inc.

*9 SA-Black BL-100PS (100%), produced by Miyoshi Kasei Inc.

*10 SP-500, produced by Toray Industries, Inc.

*11 KF-96A-20cs, produced by Shin-Etsu Chemical Co., Ltd.

*12 Cosmol 44V, produced by The Nisshin OilliO Group, Ltd.

*13 Cosmol 168ARV, produced by The Nisshin OilliO Group, Ltd.

*14 Hycol K-230, produced by Kaneda Co.,Ltd.

(Examples 9)

Pressed foundations were prepared by using components shown in Table 10-1 and Table 10-2 as follows. B was heated and dissolved. A was weighed and mixed by using Labo Milser for 30 seconds. B was added to A, and further agitated by using Labo Mixer for 5 minutes, and mixed for 30 seconds $\times$ three times by using Labo Milser. The mixture was sieved by using a 150-$\mu$m opening sieve to make the particle diameters uniform, and the mixture was then packed in a gold plate and compression-molded.

Examples 9-1 to 9-10 had better moldability than Comparative Examples 9-1 to 9-4, and also prescriptions excellent in scoopability were obtained.

Table 10-1

| | | Comparative Example 9-1 | Comparative Example 9-2 | Comparative Example 9-3 | Comparative Example 9-4 | Example 9-1 | Example 9-2 | Example 9-3 |
|---|---|---|---|---|---|---|---|---|
| | Example No. of the powder composition of the present invention described below | | | | | Example 2-1 | Example 2-1 | Example 2-1 |
| | | (wt%) | (wt%) | (wt%) | (wt%) | (wt%) | (wt%) | (wt%) |
| A | Talc, dimethicone * 1 | 21.40 | 15.40 | 21.40 | 15.40 | 21.40 | 20.90 | 18.40 |
| | Mica, dimethicone *2 | 31.00 | 24.00 | 31.00 | 24.00 | 31.00 | 30.50 | 28.00 |
| | Mica, dimethicone *3 | 21.00 | 15.00 | 21.00 | 15.00 | 21.00 | 20.00 | 18.00 |
| | Titanium oxide, dimethicone *4 | 8.00 | 8.00 | 8.00 | 8.00 | 8.00 | 8.00 | 8.00 |
| | Titanium oxide, Al stearate *5 | 3.00 | 3.00 | 3.00 | 3.00 | 3.00 | 3.00 | 3.00 |
| | Zinc oxide, methicone *6 | 2.00 | 2.00 | 2.00 | 2.00 | 2.00 | 2.00 | 2.00 |
| | Red iron oxide, dimethicone *7 | 0.40 | 0.40 | 0.40 | 0.40 | 0.40 | 0.40 | 0.40 |
| | Yellow iron oxide, dimethicone *8 | 1.00 | 1.00 | 1.00 | 1.00 | 1.00 | 1.00 | 1.00 |
| | Black iron oxide, dimethicone *9 | 0.15 | 0.15 | 0.15 | 0.15 | 0.15 | 0.15 | 0.15 |
| | Nylon-12 *10 | 1.00 | 20.00 | - | - | - | - | - |
| | PMMA particles | - | - | 1.00 | 20.00 | - | - | - |
| | Urethane particles | - | - | - | - | - | - | - |
| | The powder composition of the present invention | - | - | - | - | 1.00 | 3.00 | 10.00 |
| | Methylparaben | 0.05 | 0.05 | 0.05 | 0.05 | 0.05 | 0.05 | 0.05 |
| B | Dimethicone * 11 | 4.98 | 4.98 | 4.98 | 4.98 | 4.98 | 4.98 | 4.98 |
| | Polyglyceryl-2 tetraisostearate *12 | 3.00 | 3.00 | 3.00 | 3.00 | 3.00 | 3.00 | 3.00 |
| | Dipentaerythrityl hexahydroxystearate/ hexastearate/hexarosinate *13 | 1.00 | 1.00 | 1.00 | 1.00 | 1.00 | 1.00 | 1.00 |
| | Mineral oil *14 | 2.00 | 2.00 | 2.00 | 2.00 | 2.00 | 2.00 | 2.00 |
| | Methylparaben | 0.01 | 0.01 | 0.01 | 0.01 | 0.01 | 0.01 | 0.01 |
| | Tocopherol | 0.01 | 0.01 | 0.01 | 0.01 | 0.01 | 0.01 | 0.01 |

(continued)

| | | Comparative Example 9-1 | Comparative Example 9-2 | Comparative Example 9-3 | Comparative Example 9-4 | Example 9-1 | Example 9-2 | Example 9-3 |
|---|---|---|---|---|---|---|---|---|
| | Example No. of the powder composition of the present invention described below | | | | | Example 2-1 | Example 2-1 | Example 2-1 |
| | Total | 100.0 | 100.0 | 100.0 | 116.0 | 100.0 | 100.0 | 100.0 |
| | Sensory evaluation | C | A | C | A | C | A | A |

Table 10-2

| | | Example 9-4 | Example 9-5 | Example 9-6 | Example 9-7 | Example 9-8 | Example 9-9 | Example 9-10 |
|---|---|---|---|---|---|---|---|---|
| | Example No. of the powder composition of the present invention described below | Example 2-1 | Example 2-1 | Example 2-10 | Example 2-10 | Example 2-10 | Example 2-10 | Example 2-10 |
| | | (wt%) | (wt%) | (wt%) | (wt%) | (wt%) | (wt%) | (wt%) |
| A | Talc, dimethicone *1 | 15.40 | 13.90 | 21.40 | 20.90 | 18.40 | 15.40 | 13.90 |
| | Mica, dimethicone *2 | 24.00 | 17.00 | 31.00 | 30.50 | 28.00 | 24.00 | 17.00 |
| | Mica, dimethicone *3 | 15.00 | 13.50 | 21.00 | 20.00 | 18.00 | 15.00 | 13.50 |
| | Titanium oxide, dimethicone *4 | 8.00 | 8.00 | 8.00 | 8.00 | 8.00 | 8.00 | 8.00 |
| | Titanium oxide, Al stearate *5 | 3.00 | 3.00 | 3.00 | 3.00 | 3.00 | 3.00 | 3.00 |
| | Zinc oxide, methicone *6 | 2.00 | 2.00 | 2.00 | 2.00 | 2.00 | 2.00 | 2.00 |
| | Red iron oxide, dimethicone *7 | 0.40 | 0.40 | 0.40 | 0.40 | 0.40 | 0.40 | 0.40 |
| | Yellow iron oxide, dimethicone *8 | 1.00 | 1.00 | 1.00 | 1.00 | 1.00 | 1.00 | 1.00 |
| | Black iron oxide, dimethicone *9 | 0.15 | 0.15 | 0.15 | 0.15 | 0.15 | 0.15 | 0.15 |
| | Nylon-12 *10 | - | - | - | - | - | - | - |
| | PMMA particles | - | - | - | - | - | - | - |
| | Urethane particles | - | - | - | - | - | - | - |
| | The powder composition of the present invention | 20.00 | 30.00 | 1.00 | 3.00 | 10.00 | 20.00 | 30.00 |
| | Methylparaben | 0.05 | 0.05 | 0.05 | 0.05 | 0.05 | 0.05 | 0.05 |
| B | Dimethicone *11 | 4.98 | 4.98 | 4.98 | 4.98 | 4.98 | 4.98 | 4.98 |
| | Polyglyceryl-2 tetraisostearate *12 | 3.00 | 3.00 | 3.00 | 3.00 | 3.00 | 3.00 | 3.00 |
| | Dipentaerythrityl hexahydroxystearate/ hexastearate/hexarosinate *13 | 1.00 | 1.00 | 1.00 | 1.00 | 1.00 | 1.00 | 1.00 |
| | Mineral oil *14 | 2.00 | 2.00 | 2.00 | 2.00 | 2.00 | 2.00 | 2.00 |
| | Methylparaben | 0.01 | 0.01 | 0.01 | 0.01 | 0.01 | 0.01 | 0.01 |
| | Tocopherol | 0.01 | 0.01 | 0.01 | 0.01 | 0.01 | 0.01 | 0.01 |
| | Total | 100.0 | 100.0 | 100.0 | 100.0 | 100.0 | 100.0 | 100.0 |

(continued)

| | | Example 9-4 | Example 9-5 | Example 9-6 | Example 9-7 | Example 9-8 | Example 9-9 | Example 9-10 |
|---|---|---|---|---|---|---|---|---|
| | Example No. of the powder composition of the present invention described below | Example 2-1 | Example 2-1 | Example 2-10 | Example 2-10 | Example 2-10 | Example 2-10 | Example 2-10 |
| | Sensory evaluation | A | C | C | A | A | A | C |

Raw materials: Raw materials used in Examples and Comparative Examples shown in Table 10-1 to Table 10-2 described above are as follows.

*1 SA-Talc JA-46R, produced by Miyoshi Kasei Inc.

*2 SA-Sericite FSE, produced by Miyoshi Kasei Inc.

*3 SA-Mica Y-2300, produced by Miyoshi Kasei Inc.

*4 SA-Ttitanium CR50 (100%), produced by Miyoshi Kasei Inc.

*5 MT-100Z, produced by Tayca Corporation

*6 MZ-303S, produced by Tayca Corporation

*7 SA-Red R-516PS (100%), produced by Miyoshi Kasei Inc.

*8 SA-Yellow LL-100P (100%), produced by Miyoshi Kasei Inc.

*9 SA-Black BL-100PS(100%), produced by Miyoshi Kasei Inc.

*10 SP-500, produced by Toray Industries, Inc.

*11 KF-96A-20cs, produced by Shin-Etsu Chemical Co., Ltd.

*12 Cosmol 44V, produced by The Nisshin OilliO Group, Ltd.

*13 Cosmol 168ARV, produced by The Nisshin OilliO Group, Ltd.

*14 Hycol K-230, produced by Kaneda Co.,Ltd.

(Evaluation Using Liquid Foundation)

As cosmetics or skin topical agents using the powder compositions of the present invention, liquid foundations were prepared, and were evaluated.

Regarding the evaluation on the usability, 5 expert panelists gave scores to 4 evaluation items, namely, "adhesion", "smoothness on application", "non-stickiness", "moisturization", and the score of each evaluation item was determined using an average score of the 5 panelists. Furthermore, evaluation was determined in accordance with a 5-scale of A to D with an average value of the scores of each evaluation item.

(Score): (Evaluation)

6: Very Good
5: Good
4: Slightly Good
3: Slightly Poor
2: Poor
1: Very Poor

(Determination):(Score Average Value)

A: 5 points or more
B: 4 points or more and less than 5 points
C: 3 points or more and less than 4 points
D: less than 3 points

(Examples 10)

Liquid foundations were prepared by using components shown in Table 11-1 and Table 11-2 as follows.
A was heated (75°C) and mixed (4000 rpm) by using a homo-mixer. B was added to A, followed by heating (75°C) and mixing (4000 rpm) by using the homo-mixer. Moreover, C was added to A, followed by heating (75°C) and mixing (4000 rpm) by using the homo-mixer. D was heated to 75°C and dissolved, and was gradually added to A to be emulsified. Thereafter, the mixture was cooled down to 30°C while being agitated with a paddle.

Table 11-1

| | | Comparative Example 10-1 | Comparative Example 10-2 | Comparative Example 10-3 | Example 10-1 | Example 10-2 | Example 10-3 | Example 10-4 | Example 10-5 |
|---|---|---|---|---|---|---|---|---|---|
| | Example No. of the powder composition of the present invention described below | | | Comparative Example 2-2 | Example 2-1 | Example 2-4 | Example 2-5 | Example 2-7 | Example 2-8 |
| | | (wt%) | (wt%) | (wt%) | (wt%) | (wt%) | (wt%) | (wt%) | (wt%) |
| A | DC246 *1 | 24.44 | 24.44 | 24.44 | 24.44 | 24.44 | 24.44 | 24.44 | 24.44 |
| | Squalane | 2.04 | 2.04 | 2.04 | 2.04 | 2.04 | 2.04 | 2.04 | 2.04 |
| | ELDEW® PS-203 *2 | 0.51 | 0.51 | 0.51 | 0.51 | 0.51 | 0.51 | 0.51 | 0.51 |
| | Isotridecyl isononanoate *3 | 5.09 | 5.09 | 5.09 | 5.09 | 5.09 | 5.09 | 5.09 | 5.09 |
| | Polyglyceryl-2 diisostearate *4 | 2.04 | 2.04 | 2.04 | 2.04 | 2.04 | 2.04 | 2.04 | 2.04 |
| | PEG-10 Dimethicone *5 | 2.80 | 2.80 | 2.80 | 2.80 | 2.80 | 2.80 | 2.80 | 2.80 |
| | Dimethicone *6 | 2.29 | 2.29 | 2.29 | 2.29 | 2.29 | 2.29 | 2.29 | 2.29 |
| | Phenoxyethanol | 0.41 | 0.41 | 0.41 | 0.41 | 0.41 | 0.41 | 0.41 | 0.41 |
| | Tocopherol acetate | 0.10 | 0.10 | 0.10 | 0.10 | 0.10 | 0.10 | 0.10 | 0.10 |
| | Distearyldimonium chloride *7 | 0.10 | 0.10 | 0.10 | 0.10 | 0.10 | 0.10 | 0.10 | 0.10 |
| B | Quaternium-18 bentonite, isododecane, isopropylene carbonate *8 | 9.26 | 9.26 | 9.26 | 9.26 | 9.26 | 9.26 | 9.26 | 9.26 |
| C | Talc, dimethicone *9 | 10.56 | 10.56 | 10.56 | 10.56 | 10.56 | 10.56 | 10.56 | 10.56 |
| | Titanium oxide, dimethicone *10 | 3.70 | 3.70 | 3.70 | 3.70 | 3.70 | 3.70 | 3.70 | 3.70 |
| | Red iron oxide, dimethicone *11 | 0.19 | 0.19 | 0.19 | 0.19 | 0.19 | 0.19 | 0.19 | 0.19 |
| | Yellow iron oxide, dimethicone *12 | 1.20 | 1.20 | 1.20 | 1.20 | 1.20 | 1.20 | 1.20 | 1.20 |
| | Black iron oxide, dimethicone *13 | 0.09 | 0.09 | 0.09 | 0.09 | 0.09 | 0.09 | 0.09 | 0.09 |
| | Nylon-12 *14 | 8.00 | | | | | | | |
| | PMMA particles* 15 | | 8.00 | | | | | | |
| | The powder composition of the present invention | | | 8.00 | 8.00 | 8.00 | 8.00 | 8.00 | 8.00 |

(continued)

| | | Comparative Example 10-1 | Comparative Example 10-2 | Comparative Example 10-3 | Example 10-1 | Example 10-2 | Example 10-3 | Example 10-4 | Example 10-5 |
|---|---|---|---|---|---|---|---|---|---|
| | Example No. of the powder composition of the present invention described below | | | Comparative Example 2-2 | Example 2-1 | Example 2-4 | Example 2-5 | Example 2-7 | Example 2-8 |
| D | EDTA-2Na | 0.05 | 0.05 | 0.05 | 0.05 | 0.05 | 0.05 | 0.05 | 0.05 |
| | BG *16 | 4.63 | 4.63 | 4.63 | 4.63 | 4.63 | 4.63 | 4.63 | 4.63 |
| | Purified water | 22.50 | 22.50 | 22.50 | 22.50 | 22.50 | 22.50 | 22.50 | 22.50 |
| | Total | 100.00 | 100.00 | 100.00 | 100.00 | 100.00 | 100.00 | 100.00 | 100.00 |
| | Sensory evaluation | A | A | D | A | A | A | B | A |

Table 11-2

|  |  | Example 10-6 | Example 10-7 | Example 10-8 | Example 10-9 | Example 10-10 | Example 10-11 | Example 10-12 |
|---|---|---|---|---|---|---|---|---|
|  | Example No. of the powder composition of the present invention described below | Example 2-11 | Example 3-1 | Example 3-3 | Example 4-1 | Example 4-4 | Example 4-10 | Example 5-1 |
|  |  | (wt%) | (wt%) | (wt%) | (wt%) | (wt%) | (wt%) | (wt%) |
| A | DC246 *1 | 24.44 | 24.44 | 24.44 | 24.44 | 24.44 | 24.44 | 24.44 |
|  | Squalane | 2.04 | 2.04 | 2.04 | 2.04 | 2.04 | 2.04 | 2.04 |
|  | ELDEW® PS-203 *2 | 0.51 | 0.51 | 0.51 | 0.51 | 0.51 | 0.51 | 0.51 |
|  | Isotridecyl isononanoate *3 | 5.09 | 5.09 | 5.09 | 5.09 | 5.09 | 5.09 | 5.09 |
|  | Polyglyceryl-2 diisostearate *4 | 2.04 | 2.04 | 2.04 | 2.04 | 2.04 | 2.04 | 2.04 |
|  | PEG-10 dimethicone *5 | 2.80 | 2.80 | 2.80 | 2.80 | 2.80 | 2.80 | 2.80 |
|  | Dimethicone *6 | 2.29 | 2.29 | 2.29 | 2.29 | 2.29 | 2.29 | 2.29 |
|  | Phenoxyethanol | 0.41 | 0.41 | 0.41 | 0.41 | 0.41 | 0.41 | 0.41 |
|  | Tocopherol acetate | 0.10 | 0.10 | 0.10 | 0.10 | 0.10 | 0.10 | 0.10 |
|  | Distearyldimonium chloride *7 | 0.10 | 0.10 | 0.10 | 0.10 | 0.10 | 0.10 | 0.10 |
| B | Quaternium-18 bentonite, isododecane, isopropylene carbonate *8 | 9.26 | 9.26 | 9.26 | 9.26 | 9.26 | 9.26 | 9.26 |
| C | Talc, dimethicone *9 | 10.56 | 10.56 | 10.56 | 10.56 | 10.56 | 10.56 | 10.56 |
|  | Titanium oxide, dimethicone *10 | 3.70 | 3.70 | 3.70 | 3.70 | 3.70 | 3.70 | 3.70 |
|  | Red iron oxide, dimethicone *11 | 0.19 | 0.19 | 0.19 | 0.19 | 0.19 | 0.19 | 0.19 |
|  | Yellow iron oxide, dimethicone *12 | 1.20 | 1.20 | 1.20 | 1.20 | 1.20 | 1.20 | 1.20 |
|  | Black iron oxide, dimethicone *13 | 0.09 | 0.09 | 0.09 | 0.09 | 0.09 | 0.09 | 0.09 |
|  | Nylon-12 *14 |  |  |  |  |  |  |  |
|  | PMMA particles *15 |  |  |  |  |  |  |  |
|  | The powder composition of the present invention | 8.00 | 8.00 | 8.00 | 8.00 | 8.00 | 8.00 | 8.00 |
| D | EDTA-2Na | 0.05 | 0.05 | 0.05 | 0.05 | 0.05 | 0.05 | 0.05 |
|  | BG *16 | 4.63 | 4.63 | 4.63 | 4.63 | 4.63 | 4.63 | 4.63 |
|  | Purified water | 22.50 | 22.50 | 22.50 | 22.50 | 22.50 | 22.50 | 22.50 |

(continued)

|  |  | Example 10-6 | Example 10-7 | Example 10-8 | Example 10-9 | Example 10-10 | Example 10-11 | Example 10-12 |
|---|---|---|---|---|---|---|---|---|
|  | Example No. of the powder composition of the present invention described below | Example 2-11 | Example 3-1 | Example 3-3 | Example 4-1 | Example 4-4 | Example 4-10 | Example 5-1 |
|  | Total | 100.00 | 100.00 | 100.00 | 100.00 | 100.00 | 100.00 | 100.00 |
|  | Sensory evaluation | A | B | A | A | A | B | A |

Raw materials: Raw materials used in Examples and Comparative Examples shown in Table 11-1 to Table 11-2 described above are as follows.
*1 Cyclohexasiloxane, Cyclopentasiloxane
*2 produced by Ajinomoto Co., Inc.
*3 EMALEX INTD-139, produced by The Nisshin OilliO Group, Ltd.
*4 EMALEX DISG-2EX, produced by Nihon Emulsion Co., Ltd.
*5 KF-6017P, produced by Shin-Etsu Chemical Co., Ltd.
*6 KF-96A-6T, produced by Shin-Etsu Chemical Co., Ltd.
*7 NIKKOL CA-3475V, produced by Nikko Chemicals Co., Ltd.
*8 BENTON GEL ISD, produced by Elementis
*9 SA-Talc JA-46R, produced by Miyoshi Kasei Inc.
*10 SA-Ttitanium CR50 (100%), produced by Miyoshi Kasei Inc.
*11 SA-Red R-516PS (100%), produced by Miyoshi Kasei Inc.
*12 SA-Yellow LL-100P (100%), produced by Miyoshi Kasei Inc.
*13 SA-Black BL-100PS (100%), produced by Miyoshi Kasei Inc.
*14 SP-500, produced by Toray Industries, Inc.
*15 Matsumoto Microsphere M-100, produced by Matsumoto Yushi-Seiyaku Co., Ltd.
*16 produced by Daicel Corporation

(Examples 11)

Liquid foundations were prepared by using components shown in Table 12-1 and Table 12-2 as follows.

A was heated (75°C) and mixed (4000 rpm) by using a homo-mixer. B was added to A, followed by heating (75°C) and mixing (4000 rpm) by using the homo-mixer. Moreover, C was added to A, followed by heating (75°C) and mixing (4000 rpm) by using the homo-mixer. D was heated to 75°C and dissolved, and was gradually added to A to be emulsified. Thereafter, the mixture was cooled down to 30°C while being agitated with a paddle.

Table 12-1

| | | Comparative Example 11-1 | Comparative Example 11-2 | Comparative Example 11-3 | Comparative Example 11-4 | Example 11-1 | Example 11-2 | Example 11-3 |
|---|---|---|---|---|---|---|---|---|
| | Example No. of the powder composition of the present invention described below | | | | | Example 2-1 | Example 2-1 | Example 2-1 |
| | | (wt%) | (wt%) | (wt%) | (wt%) | (wt%) | (wt%) | (wt%) |
| A | DC246 *1 | 24.44 | 20.44 | 24.44 | 20.44 | 24.44 | 23.44 | 20.44 |
| | Squalane | 2.04 | 2.04 | 2.04 | 2.04 | 2.04 | 2.04 | 2.04 |
| | ELDEW® PS-203 *2 | 0.51 | 0.51 | 0.51 | 0.51 | 0.51 | 0.51 | 0.51 |
| | Isotridecyl isononanoate *3 | 5.09 | 5.09 | 5.09 | 5.09 | 5.09 | 5.09 | 5.09 |
| | Polyglyceryl-2 diisostearate *4 | 2.04 | 2.04 | 2.04 | 2.04 | 2.04 | 2.04 | 2.04 |
| | PEG-10 dimethicone *5 | 2.80 | 2.80 | 2.80 | 2.80 | 2.80 | 2.80 | 2.80 |
| | Dimethicone *6 | 2.29 | 2.29 | 2.29 | 2.29 | 2.29 | 2.29 | 2.29 |
| | phenoxyethanol | 0.41 | 0.41 | 0.41 | 0.41 | 0.41 | 0.41 | 0.41 |
| | Tocopherol acetate | 0.10 | 0.10 | 0.10 | 0.10 | 0.10 | 0.10 | 0.10 |
| | Distearyldimonium chloride *7 | 0.10 | 0.10 | 0.10 | 0.10 | 0.10 | 0.10 | 0.10 |
| B | Quaternium-18 bentonite, isododecane, isopropylene carbonate *8 | 9.26 | 9.26 | 9.26 | 9.26 | 9.26 | 9.26 | 9.26 |
| C | Talc, dimethicone *9 | 10.56 | 10.56 | 10.56 | 10.56 | 10.56 | 10.56 | 10.56 |
| | Titanium oxide, dimethicone *10 | 3.70 | 3.70 | 3.70 | 3.70 | 3.70 | 3.70 | 3.70 |
| | Red iron oxide, dimethicone *11 | 0.19 | 0.19 | 0.19 | 0.19 | 0.19 | 0.19 | 0.19 |
| | Yellow iron oxide, dimethicone *12 | 1.20 | 1.20 | 1.20 | 1.20 | 1.20 | 1.20 | 1.20 |
| | Black iron oxide, dimethicone *13 | 0.09 | 0.09 | 0.09 | 0.09 | 0.09 | 0.09 | 0.09 |
| | Nylon-12 *14 | 1.00 | 15.00 | | | | | |
| | PMMA particles *15 | | | 1.00 | 15.00 | | | |
| | The powder composition of the present invention | | | | | 1.00 | 3.00 | 15.00 |

EP 4 268 902 A1

| | | Comparative Example 11-1 | Comparative Example 11-2 | Comparative Example 11-3 | Comparative Example 11-4 | Example 11-1 | Example 11-2 | Example 11-3 |
|---|---|---|---|---|---|---|---|---|
| | Example No. of the powder composition of the present invention described below | | | | | Example 2-1 | Example 2-1 | Example 2-1 |
| | | (wt%) | (wt%) | (wt%) | (wt%) | (wt%) | (wt%) | (wt%) |
| D | EDTA-2Na | 0.05 | 0.05 | 0.05 | 0.05 | 0.05 | 0.05 | 0.05 |
| | BG *16 | 4.63 | 4.63 | 4.63 | 4.63 | 4.63 | 4.63 | 4.63 |
| | Purified water | 29.50 | 19.50 | 29.50 | 19.50 | 29.50 | 28.50 | 19.50 |
| | Total | 100.00 | 100.00 | 100.00 | 100.00 | 100.00 | 100.00 | 100.00 |
| | Sensory evaluation | C | A | C | A | C | A | A |

Table 12-2

| | | Example 11-4 | Example 11-6 | Example 11-7 | Example 11-8 | Example 11-9 | Example 11-11 | Example 11-12 |
|---|---|---|---|---|---|---|---|---|
| | Example No. of the powder composition of the present invention described below | Example 2-1 | Example 2-1 | Example 2-10 | Example 2-10 | Example 2-10 | Example 2-10 | Example 2-10 |
| | | (wt%) | (wt%) | (wt%) | (wt%) | (wt%) | (wt%) | (wt%) |
| A | DC246 *1 | 4.44 | 24.44 | 23.44 | 20.44 | 4.44 | 23.44 | 20.44 |
| | Squalane | 2.04 | 2.04 | 2.04 | 2.04 | 2.04 | 2.04 | 2.04 |
| | ELDEW® PS-203 *2 | 0.51 | 0.51 | 0.51 | 0.51 | 0.51 | 0.51 | 0.51 |
| | Isotridecyl isononanoate *3 | 5.09 | 5.09 | 5.09 | 5.09 | 5.09 | 5.09 | 5.09 |
| | Polyglyceryl-2 diisostearate *4 | 2.04 | 2.04 | 2.04 | 2.04 | 2.04 | 2.04 | 2.04 |
| | PEG-10 dimethicone *5 | 2.80 | 2.80 | 2.80 | 2.80 | 2.80 | 2.80 | 2.80 |
| | Dimethicone *6 | 2.29 | 2.29 | 2.29 | 2.29 | 2.29 | 2.29 | 2.29 |
| | Phenoxyethanol | 0.41 | 0.41 | 0.41 | 0.41 | 0.41 | 0.41 | 0.41 |
| | Tocopherol acetate | 0.10 | 0.10 | 0.10 | 0.10 | 0.10 | 0.10 | 0.10 |
| | Distearyldimonium chloride *7 | 0.10 | 0.10 | 0.10 | 0.10 | 0.10 | 0.10 | 0.10 |
| B | Quaternium-18 bentonite, isododecane, isopropylene carbonate *8 | 9.26 | 9.26 | 9.26 | 9.26 | 9.26 | 9.26 | 9.26 |
| C | Talc, Dimethicone *9 | 10.56 | 10.56 | 10.56 | 10.56 | 10.56 | 10.56 | 10.56 |
| | Titanium oxide, dimethicone *10 | 3.70 | 3.70 | 3.70 | 3.70 | 3.70 | 3.70 | 3.70 |
| | Red iron oxide, dimethicone *11 | 0.19 | 0.19 | 0.19 | 0.19 | 0.19 | 0.19 | 0.19 |
| | Yellow iron oxide, dimethicone *12 | 1.20 | 1.20 | 1.20 | 1.20 | 1.20 | 1.20 | 1.20 |
| | Black iron oxide, dimethicone *13 | 0.09 | 0.09 | 0.09 | 0.09 | 0.09 | 0.09 | 0.09 |
| | Nylon-12 *14 | | | | | | | |
| | PMMA particles *15 | | | | | | | |
| | The powder composition of the present invention | 40.00 | 1.00 | 3.00 | 15.00 | 40.00 | 3.00 | 15.00 |
| D | EDTA-2Na | 0.05 | 0.05 | 0.05 | 0.05 | 0.05 | 0.05 | 0.05 |
| | BG *16 | 4.63 | 4.63 | 4.63 | 4.63 | 4.63 | 4.63 | 4.63 |
| | Purified water | 10.50 | 29.50 | 28.50 | 19.50 | 10.50 | 28.50 | 19.50 |

(continued)

|  |  | Example 11-4 | Example 11-6 | Example 11-7 | Example 11-8 | Example 11-9 | Example 11-11 | Example 11-12 |
|---|---|---|---|---|---|---|---|---|
|  | Example No. of the powder composition of the present invention described below | Example 2-1 | Example 2-1 | Example 2-10 | Example 2-10 | Example 2-10 | Example 2-10 | Example 2-10 |
|  |  | (wt%) | (wt%) | (wt%) | (wt%) | (wt%) | (wt%) | (wt%) |
|  | Total | 100.00 | 100.00 | 100.00 | 100.00 | 100.00 | 100.00 | 100.00 |
|  | Sensory evaluation | A | C | C | A | A | A | C |

Raw materials: Raw materials used in Examples and Comparative Examples shown in Table 12-1 to Table 12-2 described above are as follows.
*1 Cyclohexasiloxane, Cyclopentasiloxane
*2 produced by Ajinomoto Co., Inc.
*3 EMALEX INTD-139, produced by The Nisshin OilliO Group, Ltd.
*4 EMALEX DISG-2EX, produced by Nihon Emulsion Co., Ltd.
*5 KF-6017P, produced by Shin-Etsu Chemical Co., Ltd.
*6 KF-96A-6T, produced by Shin-Etsu Chemical Co., Ltd.
*7 NIKKOL CA-3475V, produced by Nikko Chemicals Co., Ltd.
*8 BENTON GEL ISD, produced by Elementis
*9 SA-Talc JA-46R, produced by Miyoshi Kasei Inc.
*10 SA-Ttitanium CR50 (100%), produced by Miyoshi Kasei Inc.
*11 SA-Red R-516PS (100%), produced by Miyoshi Kasei Inc.
*12 SA-Yellow LL-100P (100%), produced by Miyoshi Kasei Inc.
*13 SA-Black BL-100PS (100%), produced by Miyoshi Kasei Inc.
*14 SP-500, produced by Toray Industries, Inc.
*15 Matsumoto Microsphere M-100, produced by Matsumoto Yushi-Seiyaku Co., Ltd.
*16 produced by Daicel Corporation

(Prescription Example 1)

As cosmetics or skin topical agents using the powder compositions of the present invention, lip cosmetics were prepared by using components shown in Table 13 as follows.
Components A was heated and dissolved at 105±5°C, and Component B was added to the Component A, and heated and dissolved at 90°C. Moreover, Component C was added, heated and mixed at 90°C, and dispersed by using a triple roll. Then, Component D was added. Component E was added, heated and mixed at 90°C, and then defoaming was conducted. The mixture was put in a mold at a fill temperature of 90°C, and after cooling, was loaded in a container. The lip cosmetic of Prescription Example 1 had little color unevenness, and had good color uniformity. Furthermore, the cosmetic of Prescription Example 1 did not cause perspiration, and had a good stability.

Table 13

| Lip cosmetic | | |
|---|---|---|
|  | Names of raw materials | Blending amount |
| A | Polyethylene wax | 2.8 |
|  | Triethylhexanoin | 15 |

(continued)

| Lip cosmetic | | | |
|---|---|---|---|
| | | Names of raw materials | Blending amount |
| | B | Candelilla wax | 1 |
| | | Paraffin wax | 7 |
| | | Microcrystalline wax | 6 |
| | | Hydrogenated polyisobutene | 12 |
| | | Dipentaerythrityl hexahydroxystearate/hexastearate/hexarosinate | 5 |
| | | Isotridecyl isononanoate | 10 |
| | | Trimethylolpropane triisostearate | 5 |
| | | Bis-behenyl/isostearyl/phytosteryl dimer dilinoleyl dimer dilinoleate | 3 |
| | | Phytosteryl/octyldodecyl lauroyl glutamate | 2 |
| | | Tocopherol | 0.1 |
| | | Antiseptic (chlorphenesin) | 0.3 |
| | C | Titanium oxide, mica, silica (compound powder) | 0.5 |
| | | Mica, titanium oxide (compound powder) | 2 |
| | D | Red 201 | 0.3 |
| | | Red 202 | 0.6 |
| | | The powder of Example 2-1 | 3.0 |
| | | Polyglyceryl-2 triisostearate | 3 |
| | E | Polyglyceryl-2 triisostearate | 21.4 |
| | | Total (mass%) | 100 |

(Prescription Example 2)

As cosmetics or skin topical agents using the powder compositions of the present invention, leave-on cosmetics were prepared by using components shown in Table 14 as follows.

After Component A and Component B were separately heated to 80°C and dissolved, the Component A was added to the Component B while agitating. The mixture was emulsified by using a homo-mixer (3000 rpm, 3 minutes, 80°C), followed by cooling to room temperature to obtain a leave-on cosmetic.

The leave-on cosmetic of Prescription Example 2 was excellent in moistness after the application, and had a good glossiness when applied. Furthermore, the leave-on cosmetic of Prescription Example 2 had good stability and antiseptic property.

Table 14

| | Leave-on cosmetic | |
|---|---|---|
| | Names of raw materials | Blending amount |
| A | UV absorber (ethylhexyl methoxycinnamate) | 2 |
| | Squalane | 6 |
| | Cetyl ethylhexanoate | 3 |
| | Cetanol | 2.8 |
| | Stearic acid | 2.4 |
| | Propylene glycol stearate | 1.2 |
| | Glyceryl stearate (SE) | 3.3 |
| | Polysorbate 60 | 0.5 |
| | PEG-40 stearate | 1.5 |
| | Tocopherol | 0.05 |
| | The powder of Example 2-8 | 6 |
| | Phytosteryl macadamiate | 1 |
| | Antiseptic (pentylene glycol) | 1 |
| B | BG | 5 |
| | Xanthan gum | 0.1 |
| | Antiseptic (sodium dehydroacetate) | 0.1 |
| | Water | 64.05 |
| | Total (mass%) | 100 |

(Examples 12-1 to 12-7)

Raw materials were weighed in a blending ratio (% by mass) shown in Table 15 such that the total weight was 5.0 g, and all the raw materials were simultaneously introduced into Labo Milser (TESCOM Mill & Mixer TML162). An evaluation sample was obtained by mixing the raw materials for 100 seconds (five times each for 20 seconds, the powder attached to the inner wall surface was scraped off between mixing processes).

Table 15

| | Example 12-1 | Example 12-2 | Example 12-3 | Example 12-4 | Example 12-5 | Example 12-6 | Example 12-7 |
|---|---|---|---|---|---|---|---|
| Acyllysine of Preparation Example 1 | 8 | 8 | 8 | 8 | 8 | 10 | 10 |
| Plate-shaped crystalline cellulose *1 | 28 | 28 | 28 | 28 | 28 | | |
| Starch *2 | | | | | | 80 | |
| Starch *3 | | | | | | | 80 |
| Porous spherical silica *4 | 64 | | | | | | |
| Porous spherical silica *5 | | 64 | | | | | |

(continued)

|  | Example 12-1 | Example 12-2 | Example 12-3 | Example 12-4 | Example 12-5 | Example 12-6 | Example 12-7 |
|---|---|---|---|---|---|---|---|
| Porous spherical silica *6 |  |  | 64 |  |  |  |  |
| Porous spherical silica *7 |  |  |  | 64 |  |  |  |
| Hollow spherical silica *8 |  |  |  |  | 64 |  |  |
| Hollow spherical silica *9 |  |  |  |  |  | 10 |  |
| Hollow spherical silica *10 |  |  |  |  |  |  | 10 |
| Raw materials: Raw materials used in Examples shown in Table 15 described above are as follows.<br>*1 produced by Nippon Paper Chemicals Co., Ltd.: average particle diameter 24 $\mu$m<br>*2 produced by DAITO KASEI KOGYO CO., LTD.: average particle diameter 10 $\mu$m<br>*3 produced by NIPPON STARCH CHEMICAL CO., LTD.: average particle diameter 18 $\mu$m<br>*4 produced by Miyoshi Kasei Inc.: average particle diameter 5 $\mu$m<br>*5 produced by Sunjin Co., Ltd.: average particle diameter 7 $\mu$m<br>*6 produced by Kobo Products Inc.: average particle diameter 20 $\mu$m<br>*7 produced by Kobo Products Inc.: average particle diameter 5 $\mu$m<br>*8 produced by Miyoshi Kasei Inc.: average particle diameter 7 $\mu$m<br>*9 produced by JGC Catalysts and Chemicals Ltd.: average particle diameter 4 $\mu$m<br>*10 SUZUKIYUSHI INDUSTRIAL CORPORATION: average particle diameter 6 $\mu$m | | | | | | | |

(Prescription Examples 3 to 9)

Loose powders were prepared by using components shown in Table 16 as follows.
Component A was mixed, and finely pulverized by using a fine pulverizer. Component B was introduced into the component A, followed by agitating and mixing. A mixture of Component C was added, followed by agitating and mixing. Moreover, a mixture of Component D was added, followed by agitating and mixing to obtain a loose powder.
The loose powders of Prescription Examples 3 to 9 were excellent in slipperiness, adhesion, and uniformity when applied, and were also excellent in moistness and soft focus effect after application.

Table 16

| | Loose powder | | | | | | | |
|---|---|---|---|---|---|---|---|---|
| | Names of raw materials | Prescription Example 3 | Prescription Example 4 | Prescription Example 5 | Prescription Example 6 | Prescription Example 7 | Prescription Example 8 | Prescription Example 9 |
| | | Blending amount | Blending amount | Blending amount | Blending amount | Blending amount | Blending amount | Blending amount |
| A | Mica | 38.8 | 38.8 | 38.8 | 38.8 | 38.8 | 38.8 | 38.8 |
| | Iron oxide (Yellow type) | 1 | 1 | 1 | 1 | 1 | 1 | 1 |
| | Iron oxide (Red type) | 0.7 | 0.7 | 0.7 | 0.7 | 0.7 | 0.7 | 0.7 |
| | Iron oxide (Black type) | 0.25 | 0.25 | 0.25 | 0.25 | 0.25 | 0.25 | 0.25 |
| | Mica, bismuth oxychloride, iron oxide (Yellow type) | 1 | 1 | 1 | 1 | 1 | 1 | 1 |
| | Mica, bismuth oxychloride, iron oxide (Red type) | 0.5 | 0.5 | 0.5 | 0.5 | 0.5 | 0.5 | 0.5 |
| | Mica, bismuth oxychloride, iron oxide (Black type) | 0.25 | 0.25 | 0.25 | 0.25 | 0.25 | 0.25 | 0.25 |
| | The powder of Example 12-1 | 30 | | | | | | |
| | The powder of Example 12-2 | | 30 | | | | | |

(continued)

| | Loose powder | | | | | | | |
|---|---|---|---|---|---|---|---|---|
| | Names of raw materials | Prescription Example 3 | Prescription Example 4 | Prescription Example 5 | Prescription Example 6 | Prescription Example 7 | Prescription Example 8 | Prescription Example 9 |
| | | Blending amount | Blending amount | Blending amount | Blending amount | Blending amount | Blending amount | Blending amount |
| A | The powder of Example 12-3 | | | 30 | | | | |
| | The powder of Example 12-4 | | | | 30 | | | |
| | The powder of Example 12-5 | | | | | 30 | | |
| | The powder of Example 12-6 | | | | | | 30 | |
| | The powder of Example 12-7 | | | | | | | 30 |
| | Titanium oxide, aluminum hydroxide, stearic acid | 3 | 3 | 3 | 3 | 3 | 3 | 3 |
| | Carnauba wax, calcium silicate | 2 | 2 | 2 | 2 | 2 | 2 | 2 |
| B | Rice bran wax | 15 | 15 | 15 | 15 | 15 | 15 | 15 |
| C | Dimethicone (50cs type) | 2 | 2 | 2 | 2 | 2 | 2 | 2 |
| | Caprylyl glycol, phenoxyethanol, hexylene glycol | 0.5 | 0.5 | 0.5 | 0.5 | 0.5 | 0.5 | 0.5 |
| D | Mica, iron oxide (Brown type) | 5 | 5 | 5 | 5 | 5 | 5 | 5 |
| | Total (mass%) | 100 | 100 | 100 | 100 | 100 | 100 | 100 |

(Prescription Example 10)

An eye makeup cosmetic was prepared by using components shown in Table 17 as follows.

A component was mixed for 10 minutes, and then B component was added to the A component, followed by further mixing for 20 minutes. C component was mixed for 10 minutes by using a mixer (FM 10C/I manufactured by Nippon Coke & Engineering Co., Ltd.), and then added to the mixture of A component+B component, followed by further mixing for 5 minutes. The mixture thus obtained was packed in a container, and was compressed by using a press machine to obtain a target eye makeup cosmetic.

The eye makeup cosmetic of Prescription Example 4 was excellent in pearlescence, gloss, shine, and color production after application.

Table 17

| Eye-makeup cosmetic | | |
|---|---|---|
| | Names of raw materials | Blending amount |
| A | Boron nitride | 11.7 |
| | Talc | 10 |
| | Myristic acid Mg | 5 |
| | Silica | 5 |
| | Iron oxide (Red) | 1 |
| | Iron oxide (Yellow) | 0.3 |
| | The powder of Example 2-1 | 2 |
| B | Caprylic/capric triglyceride | 8 |
| C | Calcium sodium borosilicate | 5 |
| | Synthetic fluorphlogopite, titanium oxide, iron oxide, tin oxide | 10 |
| | Mica, titanium oxide, iron oxide | 10 |
| | Mica, titanium oxide, iron oxide, silica, tin oxide | 8 |
| | Synthetic Fluorphlogopite, titanium oxide, carmine | 1 |
| | Mica, titanium oxide, iron oxide, chromium oxide | 8 |
| | Calcium aluminum borosilicate, titanium oxide, silica, tin oxide, dimethicone crosspolymer | 5 |
| | Mica, titanium oxide, iron oxide, ferric ferrocyanide | 3 |
| | The powder obtained in Example 2-1 | 7 |
| | Total (mass%) | 100 |

**Claims**

1. A powder composition comprising:

   (A) acyllysine; and
   (B) cellulose and/or starch, a median diameter of a volume-based distribution of the (A) acyllysine being 9 $\mu$m or less,

   wherein

   a content of the (A) is 2 to 40% by mass,
   a content of the (B) is 3 to 95% by mass, and
   a total amount of the (A) and the (B) is 10% by mass or more, in a case where the entire powder composition is considered as 100% by mass.

2. A powder composition comprising:

    (A) acyllysine; and
    (B) cellulose and/or starch,

wherein

    a content of the (A) is 2 to 40% by mass,
    a content of the (B) is 3 to 95% by mass, and
    a total amount of the (A) and the (B) is 10% by mass or more, in a case where the entire powder composition is considered as 100% by mass,
    and wherein
    a water repellent time using an aqueous solution of 35% by mass of ethanol is 30 minutes or more, wherein the water repellent time using the aqueous solution of 35% by mass of ethanol is a time taken for all the powder composition to sediment when 5 g of the aqueous solution of 35% by mass of ethanol is put in a 10-mL glass vial, and 30 mg of the powder composition is disposed on a liquid surface.

3. A powder composition comprising:

    (A) acyllysine; and
    (B) cellulose and/or starch,

wherein

    a content of the (A) is 2 to 40% by mass,
    a content of the (B) is 3 to 95% by mass, and
    a total amount of the (A) and the (B) is 10% by mass or more, in a case where the entire powder composition is considered as 100% by mass,
    and wherein
    a water repellent time using an aqueous solution of 30% by mass of ethanol is 60 minutes or more, wherein the water repellent time using the aqueous solution of 30% by mass of ethanol is a time taken for all the powder composition to sediment when 5 g of the aqueous solution of 30% by mass of ethanol is put in a 10-mL glass vial, and 30 mg of the powder composition is disposed on a liquid surface.

4. The composition according to any one of claims 1 to 3, wherein the (B) contains plate-shaped cellulose.

5. The composition according to claim 4, comprising 5 to 65% by mass of the plate-shaped cellulose in the case where the entire powder composition is considered as 100% by mass.

6. The composition according to any one of claims 1 to 5, comprising 5 to 90% by mass of starch in the case where the entire powder composition is considered as 100% by mass.

7. The composition according to any one of claims 1 to 6, wherein the (B) contains spherical cellulose.

8. The composition according to claim 7, comprising 5 to 95% by mass of the spherical cellulose in the case where the entire powder composition is considered as 100% by mass.

9. The composition according to any one of claims 1 to 8, further comprising (C) silica.

10. The composition according to claim 9, wherein

    the content of the (A) is 2 to 30% by mass,
    the content of the (B) is 3 to 65% by mass, and
    a content of the (C) is 30 to 90% by mass, in the case where the entire powder composition is considered as 100% by mass.

11. The composition according to any one of claims 1 to 10, wherein the cellulose is crystalline cellulose.

**12.** The composition according to any one of claims 1 to 11, further comprising (D) lysine or a salt thereof.

**13.** The composition according to claim 12, wherein a content of the (D) is 0.0001 to 1% by mass in the case where the entire powder composition is considered as 100% by mass.

**14.** The composition according to any one of claims 1 to 13, wherein a median diameter of a number-based distribution of the acyllysine is 4 $\mu$m or less.

**15.** The composition according to any one of claims 1 to 14, wherein the median diameter of the volume-based distribution of the acyllysine is 6 $\mu$m or less.

**16.** The composition according to any one of claims 1 to 15, wherein the median diameter of the number-based distribution of the acyllysine is 2 $\mu$m or less.

**17.** The composition according to any one of claims 1 to 16, wherein the median diameter of the volume-based distribution of the acyllysine is 4 $\mu$m or less.

**18.** The composition according to any one of claims 1 to 17, wherein a bulk density of the acyllysine is 0.38 g/mL or less.

**19.** A cosmetic or skin topical agent comprising the composition according to claims 1 to 18.

**20.** A method for producing the composition according to any one of claims 1 to 18, comprising the steps of:

introducing (A) acyllysine and (B) cellulose and/or starch; and
mixing.

**21.** A method for producing the composition according to any one of claims 9 to 18, comprising the steps of:

introducing (A) acyllysine, (B) cellulose and/or starch, and (C) silica; and
mixing.

**22.** The production method according to claim 20 or 21, wherein the mixing includes mixing through dry mixing.

**23.** The production method according to any one of claims 20 to 22, wherein the mixing includes mixing for 120 minutes or less by using a mixer.

## INTERNATIONAL SEARCH REPORT

| International application No. |
|---|
| **PCT/JP2021/048957** |

**A. CLASSIFICATION OF SUBJECT MATTER**

*A61Q 1/00*(2006.01)i; *A61K 8/02*(2006.01)i; *A61K 8/44*(2006.01)i; *A61K 8/73*(2006.01)i
FI:   A61K8/73; A61K8/44; A61K8/02; A61Q1/00

According to International Patent Classification (IPC) or to both national classification and IPC

**B. FIELDS SEARCHED**

Minimum documentation searched (classification system followed by classification symbols)

A61Q1/00; A61K8/02; A61K8/44; A61K8/73

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-180088 A (NOEVIR CO., LTD.) 05 November 2020 (2020-11-05)<br>claim 1, paragraphs [0014], [0016]-[0023] | 1-23 |
| Y | WO 01/14317 A1 (AJINOMOTO CO., INC.) 01 March 2001 (2001-03-01)<br>claims 1-9, p. 8, lines 4-30, table 1 | 1-23 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **10 February 2022** | **08 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/048957**

| Patent document cited in search report | | | Publication date (day/month/year) | Patent family member(s) | | | Publication date (day/month/year) |
|---|---|---|---|---|---|---|---|
| JP | 2020-180088 | A | 05 November 2020 | (Family: none) | | | |
| WO | 01/14317 | A1 | 01 March 2001 | US | 6555708 | B1 | |
| | | | | claims 1-9, table 1 | | | |
| | | | | EP | 1207150 | A1 | |
| | | | | CN | 1370142 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020097552 A
- JP 2020050840 A
- JP 2020152851 A
- WO 2020262367 A
- WO 01014317 A